# EUROPEAN PATENT SPECIFICATION

(11) **EP 4 153 284 B1**
(45) Date of publication and mention of the grant of the patent: **03.09.2025**
(21) Application number: 21809649.3
(22) Date of filing: 29.03.2021
(51) Int. Cl.: A61M 16/06

(54) **PATIENT CONTACTING MEMBER CONFIGURED TO RELEASABLY ENGAGE A GAS DELIVERY TUBE WHICH FORMS PART OF A POSITIONING AND STABILISING STRUCTURE FOR A PATIENT INTERFACE**
PATIENTENKONTAKTELEMENT ZUM LÖSBAREN EINGRIFF MIT EINEM GASZUFUHRSCHLAUCH, DER TEIL EINER POSITIONIERUNGS- UND STABILISIERUNGSSTRUKTUR FÜR EINE PATIENTENSCHNITTSTELLE IST
ÉLÉMENT DE CONTACT AVEC UN PATIENT CONÇU POUR VENIR EN PRISE AMOVIBLE AVEC UN TUBE D'ADMINISTRATION DE GAZ QUI FAIT PARTIE D'UNE STRUCTURE DE POSITIONNEMENT ET DE STABILISATION POUR UNE INTERFACE PATIENT

(30) Priority: 21.05.2020 AU 2020901639
(43) Date of publication of application: 29.03.2023
(73) Proprietor: ResMed Pty Ltd, Bella Vista, New South Wales 2153 (AU)
(72) Inventor: KOOIJ, Michiel, Bella Vista, New South Wales 2153 (AU); BARLOW, Adam Francis, Bella Vista, New South Wales 2153 (AU); ORMROD, Joseph Samuel, Bella Vista, New South Wales 2153 (AU); SCHEINER, Rupert Christian, Bella Vista, New South Wales 2153 (AU); WAGNER, Stewart Joseph, Bella Vista, New South Wales 2153 (AU); TRUSCOTT, Michael Kenneth, Bella Vista, New South Wales 2153 (AU)
(74) Representative: Vossius & Partner Patentanwälte Rechtsanwälte mbB
(86) International application number: PCT/AU2021/050275
(87) International publication number: WO 2021/232087

(56) References cited:
- WO-A1-2020/000033
- WO-A1-2020/037360
- US-A1- 2003 034 030
- US-A1- 2006 196 511
- US-A1- 2008 047 560
- US-A1- 2008 047 560
- US-A1- 2008 295 835
- US-A1- 2009 032 018
- US-A1- 2009 032 018
- US-A1- 2009 217 929
- US-A1- 2012 285 471
- US-A1- 2018 099 113
- US-A1- 2019 022 343
- US-A1- 2019 022 343
- US-A1- 2019 232 013
- US-A1- 2019 240 438
- US-A1- 2019 344 036
- US-A1- 2020 114 107
- US-A1- 2020 114 107
- US-A1- 2020 114 109
- US-B2- 10 279 138

## Description

### 1 BACKGROUND OF THE TECHNOLOGY

### 1.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 1.2 DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

### 1.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 1.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 1.2.2.2 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 1.2.2.2.1 Seal-forming structure

Patient interfaces may include a seal-forming structure. Since it is in direct contact with the patient's face, the shape and configuration of the seal-forming structure can have a direct impact the effectiveness and comfort of the patient interface.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFT^{™} nasal pillows mask, SWIFT^{™} II nasal pillows mask, SWIFT^{™} LT nasal pillows mask, SWIFT^{™} FX nasal pillows mask and MIRAGE LIBERTY^{™} full-face mask. The following patent applications, assigned to ResMed Limited, describe examples of nasal pillows masks: International Patent Application WO2004/073,778 (describing amongst other things aspects of the ResMed Limited SWIFT^{™} nasal pillows), US Patent Application 2009/0044808 (describing amongst other things aspects of the ResMed Limited SWIFT^{™} LT nasal pillows); International Patent Applications WO 2005/063,328 and WO 2006/130,903 (describing amongst other things aspects of the ResMed Limited MIRAGE LIBERTY^{™} full-face mask); International Patent Application WO 2009/052,560 (describing amongst other things aspects of the ResMed Limited SWIFT^{™} FX nasal pillows).

### 1.2.2.2.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

One form of a positioning and stabilising structure comprises a pair of gas delivery tubes to receive the flow of air from a connection port on top of the patient's head and to deliver the flow of air to the entrance of the patient's airways via the seal-forming structure. In examples the gas delivery tubes may be made from silicone.

It may be necessary to manufacture this form of positioning and stabilising structure in a number of different sizes in order to accommodate the full range of patient head sizes.

Some positioning and stabilising structures of the type described immediately above may be made from silicone. Since some patients may dislike the feel of the silicone against their skin, some positioning and stabilising structures of the prior art have been made with permanently attached textile sleeves covering at least part of the gas delivery tubes. However, such sleeves may make it difficult to inspect the conduit to confirm its cleanliness. It may also be difficult to properly clean the gas delivery tube with the sleeve in place.

### 1.2.2.3 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

### 1.2.2.4 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 1.2.2.5 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air. Humidifiers therefore often have the capacity to heat the flow of air was well as humidifying it.

### 1.2.2.6 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

US 2006/196511 A1 discloses a headgear for noninvasive ventilation interface made up of a nasal cannula.

US 2009/032018 A1 discloses a system adapted to provide a flow of gas to an airway of a patient.

US 2003/034030 A1 discloses a device designed to comfortably and efficiently maintain medical tubing in place on the head of a hospitalized patient.

US 2012/285471 A1 discloses a device designed to eliminate the discomfort and irritation behind the ears caused from oxygen supply line tubes by having the oxygen supply line tube wrapped on the holster and off the ears and securely in place.

US 2008/047560 A1 discloses a patient interface that includes a sealing arrangement adapted to provide an effective seal with the patient's nose, an inlet conduit arrangement adapted to deliver breathable gas to the sealing arrangement, and a cover that substantially encloses the sealing arrangement and/or the inlet conduit arrangement.

US 2020/114107 A1 discloses a patient interface that comprises a support structure and a seal-forming structure.

US 10,279,138 B2 discloses an air delivery conduit that includes first and second conduit portions that cooperate to form the conduit, each conduit portion including an inner layer of a film laminate that forms an interior surface of the conduit and an outer layer of a textile that forms an exterior surface of the conduit.

US 2019/022343 A1 discloses a positioning and stabilising structure to hold a seal-forming structure in a therapeutically effective position on a head of a patient.

### SUMMARY OF THE INVENTION

The present invention provides a patient contacting member as defined in claim 1. Further preferred embodiments of the invention are defined in the dependent claims.

### 2 BRIEF SUMMARY OF THE TECHNOLOGY

Reference will be made in the following description to various examples or aspects of the present technology. Examples or aspects disclosed herein which do not fall under the scope of the appended claims do not form part of the present invention, but are useful for understanding the principles of the invention. The scope of the present invention is defined by the appended claims.

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

One form of the present technology comprises a patient contacting member comprising an elongate sleeve portion that is releasably engageable with a gas delivery tube which forms part of a positioning and stabilising structure for a patient interface.

Another form of the present technology comprises a system for positioning and stabilising a patient interface comprising a positioning and stabilising structure comprising at least one gas delivery tube, the system comprising a plurality of patient contacting members which are releasably engageable with the gas delivery tube.

One form of the present technology comprises a patient contacting member configured to releasably engage a gas delivery tube which forms part of a positioning and stabilising structure for a patient interface, the patient contacting member comprising an elongate sleeve portion which is engageable with the gas delivery tube and a strap configured to engage a back of a patient's head, in use.

In examples:
a. the elongate sleeve portion has a length and a width and the length is more than twice the width;
b. the length is more than four times the width;
c. the elongate sleeve portion is configured to be located adjacent an interfacing structure of the patient interface in use;
d. the patient contacting member comprises two elongate sleeve portions;
e. the strap comprises two strap portions and a connector means which connects the strap portions to each other;
f. the connector means allows a length of the strap to be adjusted;
g. the strap comprises an elastically extendible portion;
h. the strap is releasably connectable to the elongate sleeve portion;
i. the elongate sleeve portion comprises a receiving means comprising an opening for receiving the strap;
j. the elongate sleeve portion comprises a plurality of receiving means spaced apart along the elongate sleeve portion;
k. the or each receiving means comprises a tab provided with a slot to receive the strap;
l. the elongate sleeve portion comprises a rigidized portion;
m. the rigidized portion is curved;
n. the patient contacting member comprises a further strap or strap portion configured to pass around the back of the patient's neck, in use;
o. the elongate sleeve portion has a patient contacting side and a non-patient contacting side, wherein the patient contacting side contacts at least the patient's cheek region, in use;
p. the elongate sleeve portion has a window portion in the non-patient contacting side;
q. the window portion comprises an opening in the patient contacting member;
r. the window portion comprises a first side and an opposite second side, wherein an elasticated cord is engaged with the first side and the second side;
s. the first side is provided with a plurality of first loops and the second side is provided with a plurality of second loops, wherein the elasticated cord extends through a plurality of the first loops and a plurality of the second loops;
t. a portion of the non-patient contacting side comprises a mesh; and/or
u. the window portion comprises a transparent material.

Another aspect of certain forms of the present technology is a system for positioning and stabilising a patient interface comprising:
a positioning and stabilising structure comprising at least one gas delivery tube; and
a plurality of patient contacting members, each patient contacting member comprising an elongate sleeve portion which is interchangeably engageable with the gas delivery tube and a strap configured to engage a back of a patient's head, in use,
wherein a length of the elongate sleeve portion of a first of the plurality of patient contacting members is different to a length of the elongate sleeve portion of a second of the plurality of the patient contacting members.

Another aspect of certain forms of the present technology is a system for positioning and stabilising a patient interface comprising:
a positioning and stabilising structure comprising at least one gas delivery tube; and
a plurality of patient contacting members, each patient contacting member comprising an elongate sleeve portion which is interchangeably engageable with the gas delivery tube and a strap configured to engage a back of a patient's head, in use,
wherein each patient contacting member comprises a rigidized portion which is curved along its length, and
wherein a first of the plurality of the patient contacting members has a different arc length to a second of the patient contacting members.

Another aspect of certain forms of the present technology is a patient contacting member configured to releasably engage a gas delivery tube for a patient interface, wherein the patient contacting member comprises a patient contacting portion and a resiliently flexible clipping portion configured to releasably engage the gas delivery tube.

In embodiments:
a. the resiliently flexible clipping portion extends along substantially an entire length of the patient contacting portion;
b. the resiliently flexible clipping portion defines a slot which extends along a length of the resiliently flexible clipping portion;
c. the gas delivery tube forms part of a positioning and stabilising structure for the patient interface;
d. the patient contacting member comprises a foam layer; and/or
e. the patient contacting member comprises a textile layer on a patient-contacting side of the patient contacting member.

Another aspect of certain forms of the present technology is a patient contacting member configured to releasably engage a gas delivery tube structure for a patient interface, wherein the patient contacting member comprises a patient contacting portion and an engaging portion which defines a slot along a length thereof, wherein the slot is shaped and configured such that the gas delivery tube can be inserted into the patient contacting member through the slot.

In embodiments:
a. the engaging portion comprises a resiliently flexible clipping portion;
b. the gas delivery tube forms part of a positioning and stabilising structure for the patient interface;
c. the patient contacting member comprises a foam layer; and/or
d. the patient contacting member comprises a textile layer on a patient-contacting side of the patient contacting member.

Another aspect of certain forms of the present technology is a system for positioning and stabilising a patient interface comprising:
a positioning and stabilising structure comprising at least one gas delivery tube; and
a plurality of patient contacting members, each patient contacting configured to releasably engage the gas delivery tube, wherein each patient contacting member comprises a patient contacting portion and a resiliently flexible clipping portion configured to releasably engage the gas delivery tube;
wherein a length of the patient contacting portion of a first of the plurality of patient contacting members is different to a length of the patient contacting portion of a second of the plurality of the patient contacting members.

Another aspect of certain forms of the present technology is a system for positioning and stabilising a patient interface comprising:
a positioning and stabilising structure comprising at least one gas delivery tube; and
a plurality of patient contacting members, each patient contacting member configured to releasably engage the gas delivery tube, wherein each patient contacting member comprises a patient contacting portion and a resiliently flexible clipping portion configured to releasably engage the gas delivery tube;
wherein each patient contacting member comprises a rigidized portion which is curved along its length, and
wherein a first of the plurality of the patient contacting members has a different arc length to a second of the patient contacting members.

Another aspect of certain forms of the present technology is a gas delivery tube assembly for a patient interface, the assembly comprising a gas delivery tube having a patient-facing side and a non patient-facing side, the assembly further comprising a liner member releasably connectable to the patient-facing side of the gas delivery tube, wherein one of the patient-facing side of the gas delivery tube and the liner member comprises at least one section of unbroken loop material and the other of the patient-facing side of the gas delivery tube and the liner member comprises at least one section of hook material configured to releasably engage the unbroken loop material.

In embodiments:
a. the gas delivery tube as forms part of a positioning and stabilising structure for the patient interface;
b. the liner member comprises a foam layer;
c. the liner member comprises a textile layer on a patient-contacting side of the liner member;
d. one of the patient-facing side of the gas delivery tube and the liner member comprises a plurality of sections of unbroken loop material and/or hook material;
e. both the patient-facing side of the gas delivery tube and the liner member comprise a plurality of sections of unbroken loop material and/or hook material; and/or
f. the liner member has a thickness of at least 2mm.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 3 BRIEF DESCRIPTION OF THE DRAWINGS

The present technology is illustrated by way of example, and not by way of limitation, in the figures of the accompanying drawings, in which like reference numerals refer to similar elements including:

### 3.1 RESPIRATORY THERAPY SYSTEMS

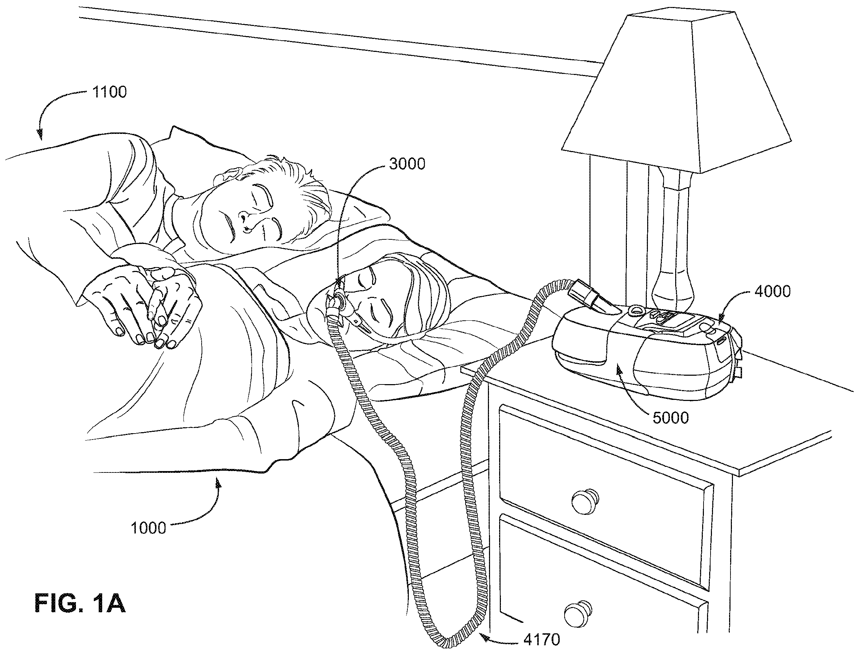

Fig. 1A shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.

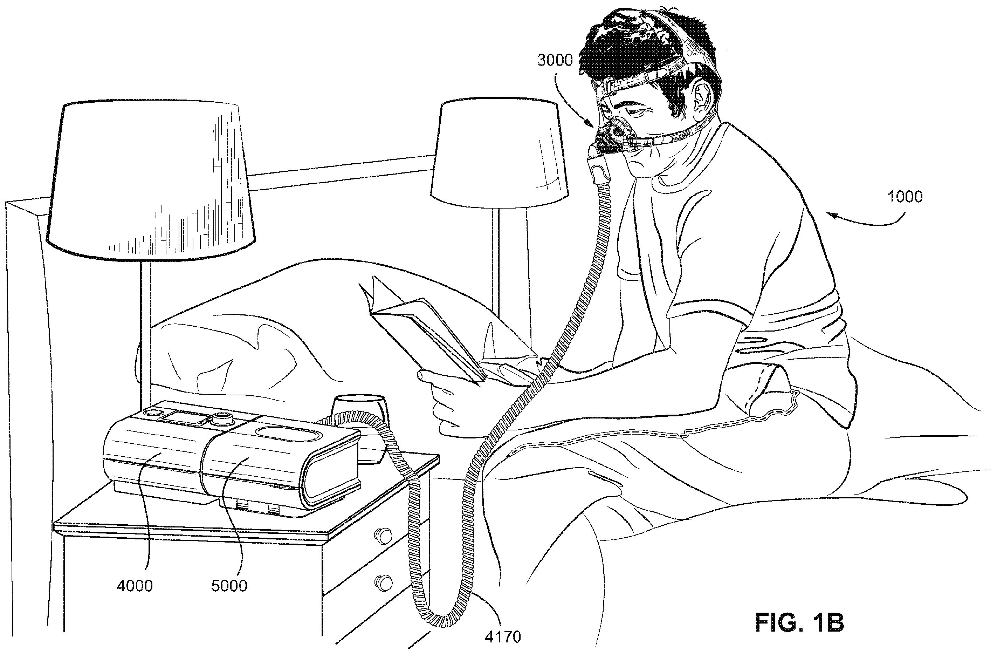

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

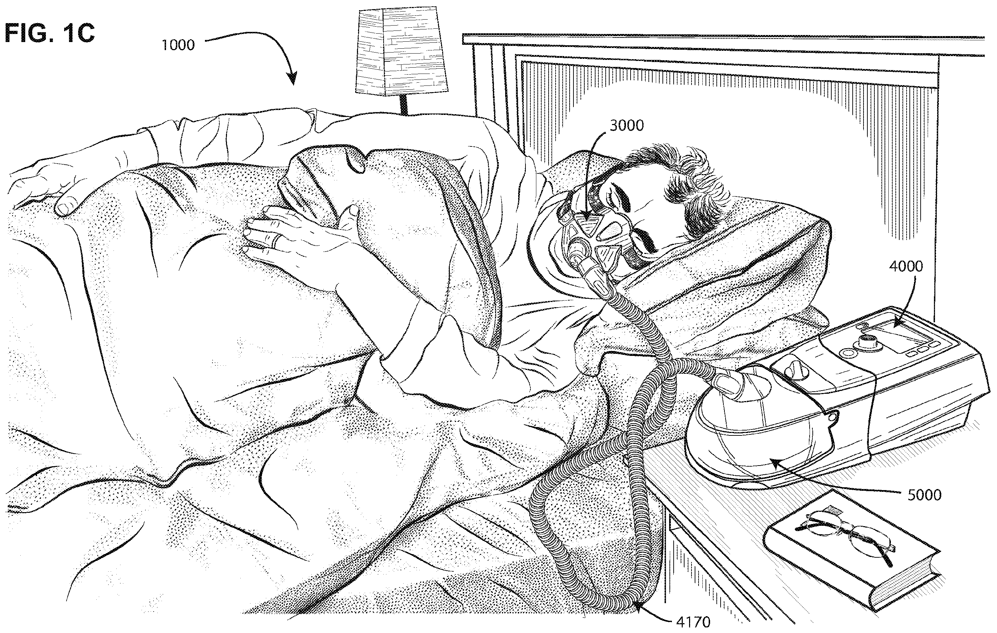

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 3.2 RESPIRATORY SYSTEM AND FACIAL ANATOMY

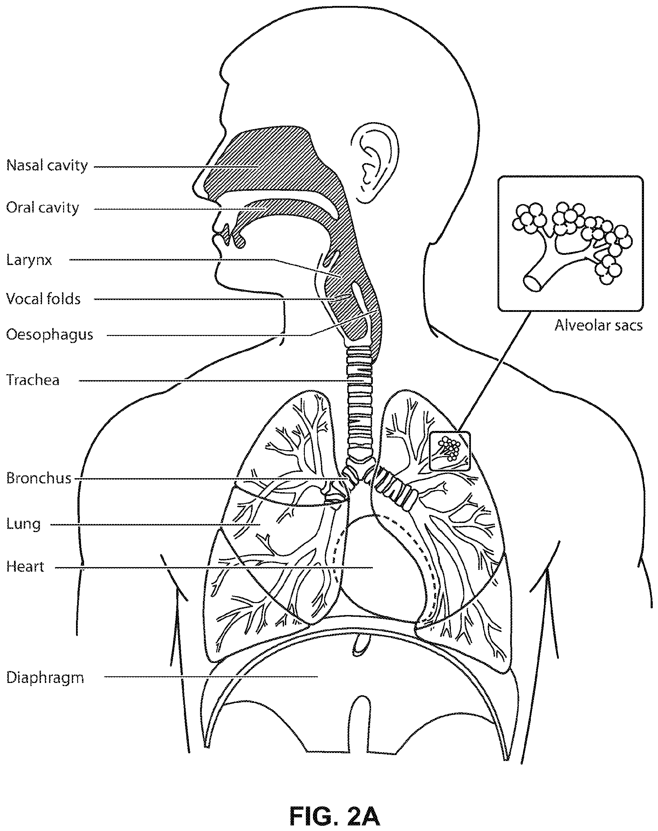

Fig. 2A shows an overview of a human respiratory system including the nasal and oral cavities, the larynx, vocal folds, oesophagus, trachea, bronchus, lung, alveolar sacs, heart and diaphragm.

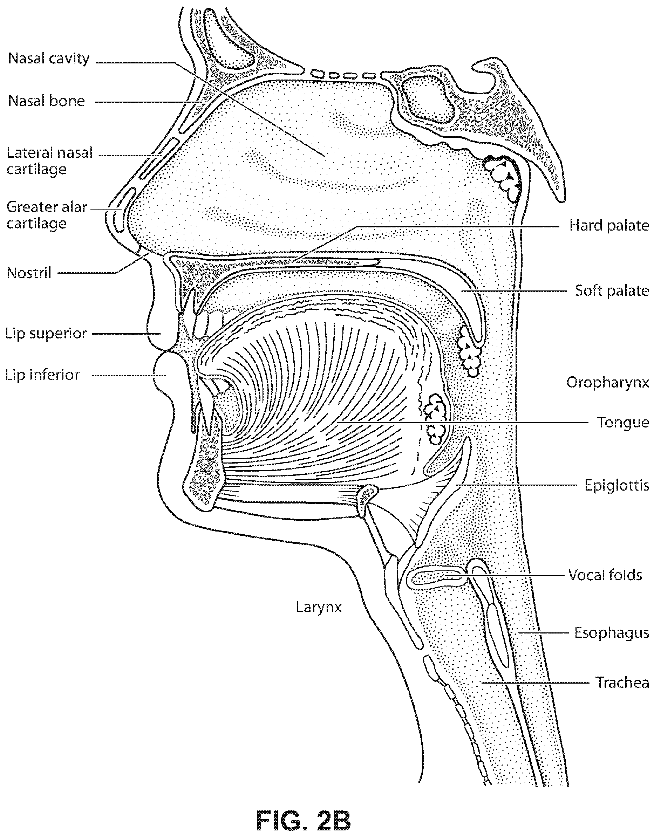

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

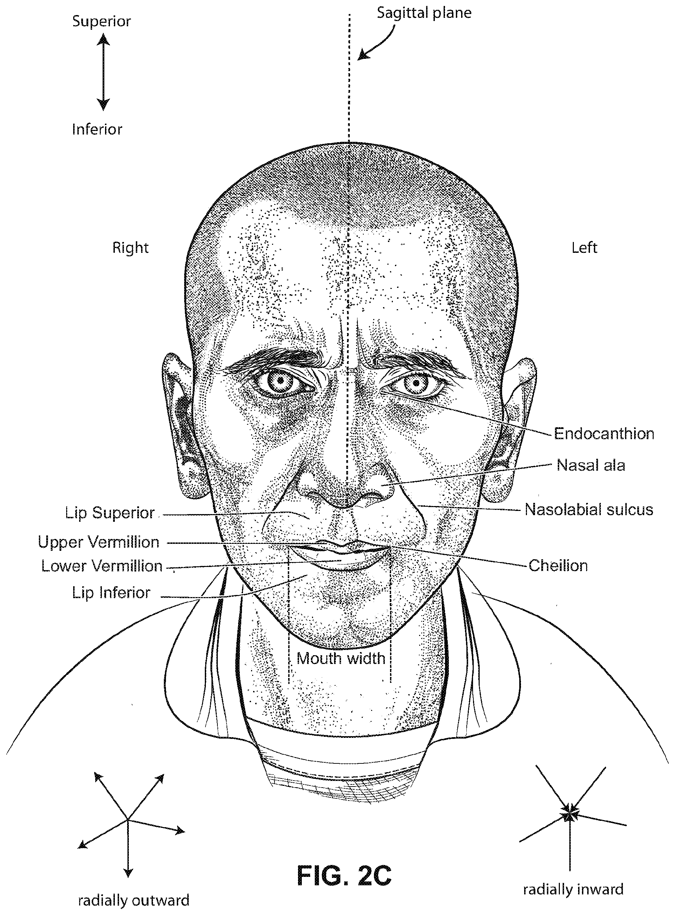

Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

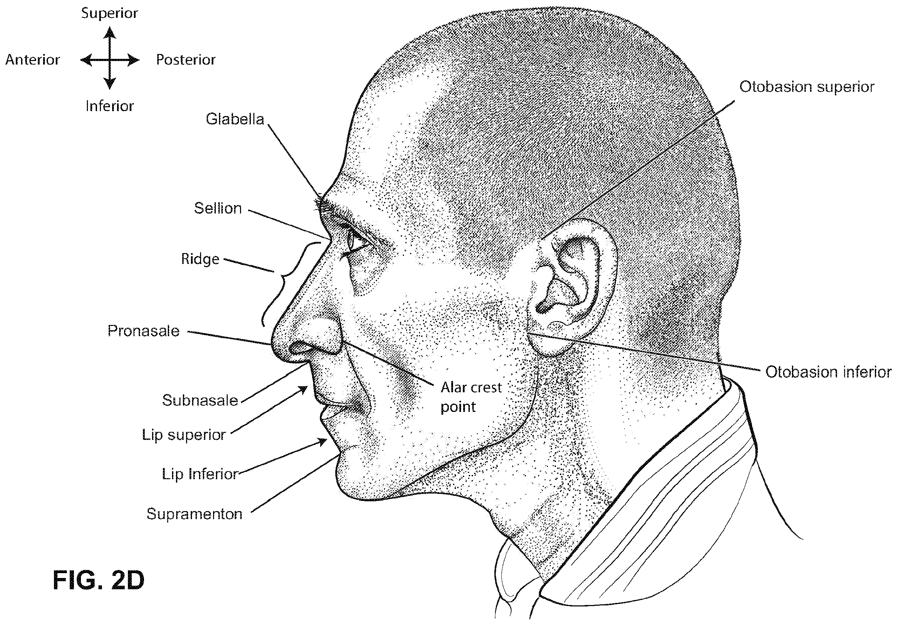

Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

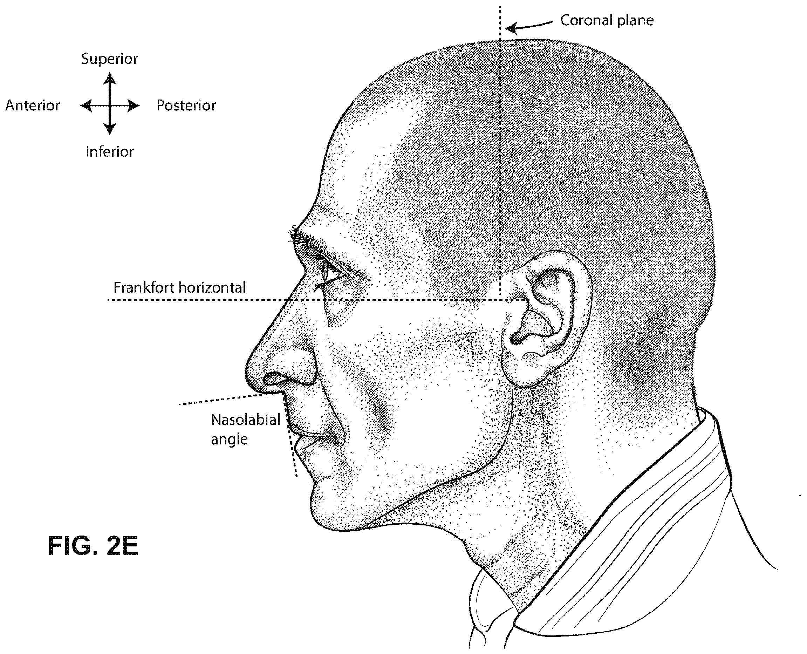

Fig. 2E is a further side view of a head. The approximate locations of the Frankfort horizontal and nasolabial angle are indicated. The coronal plane is also indicated.

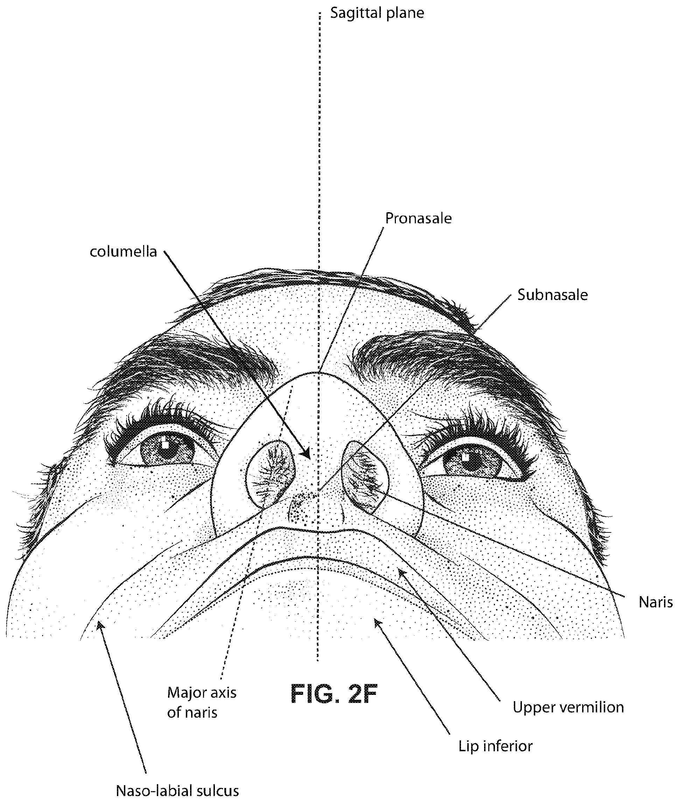

Fig. 2F shows a base view of a nose with several features identified including naso-labial sulcus, lip inferior, upper Vermilion, naris, subnasale, columella, pronasale, the major axis of a naris and the midsagittal plane.

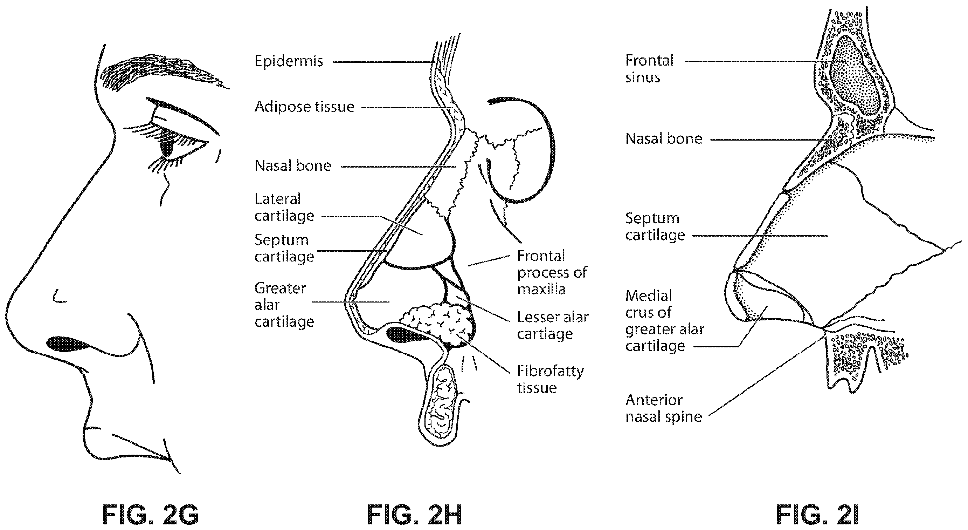

Fig. 2G shows a side view of the superficial features of a nose.
Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
Fig. 2I shows a medial dissection of a nose, approximately several millimeters from the midsagittal plane, amongst other things showing the septum cartilage and medial crus of greater alar cartilage.

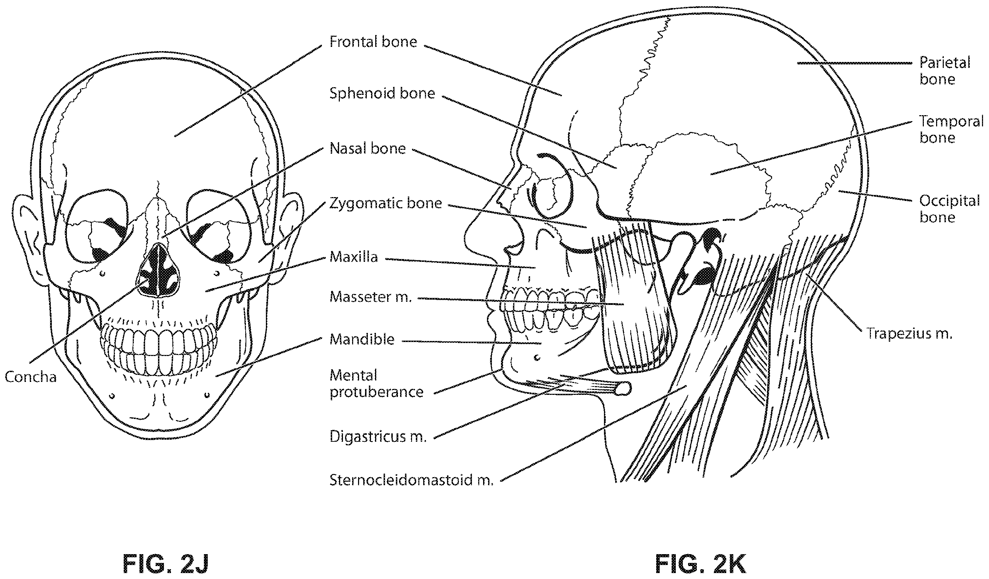

Fig. 2J shows a front view of the bones of a skull including the frontal, nasal and zygomatic bones. Nasal concha are indicated, as are the maxilla, and mandible.
Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

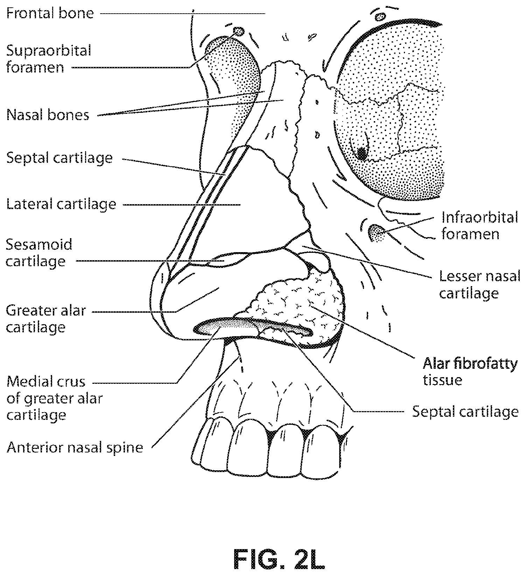

Fig. 2L shows an anterolateral view of a nose.

### 3.3 PATIENT INTERFACE

Fig. 3A shows a patient interface in the form of a nasal mask in accordance with one form of the present technology.
Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
Fig. 3D shows a schematic of a cross-section through a structure at a point. An outward normal at the point is indicated. The curvature at the point has a value of zero.
Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
Fig. 3F shows a schematic of a cross-section through a structure at a point. An outward normal at the point is indicated. The curvature at the point has a negative sign, and a relatively large magnitude when compared to the magnitude of the curvature shown in Fig. 3E.
Fig. 3G shows a cushion for a mask that includes two pillows. An exterior surface of the cushion is indicated. An edge of the surface is indicated. Dome and saddle regions are indicated.
Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.
Fig. 3I shows the surface of a structure, with a one dimensional hole in the surface. The illustrated plane curve forms the boundary of a one dimensional hole.
Fig. 3J shows a cross-section through the structure of Fig.3I. The illustrated surface bounds a two dimensional hole in the structure of Fig. 3I.
Fig. 3K shows a perspective view of the structure of Fig. 3I, including the two dimensional hole and the one dimensional hole. Also shown is the surface that bounds a two dimensional hole in the structure of Fig. 3I.
Fig. 3L shows a mask having an inflatable bladder as a cushion.
Fig. 3M shows a cross-section through the mask of Fig. 3L, and shows the interior surface of the bladder. The interior surface bounds the two dimensional hole in the mask.
Fig. 3N shows a further cross-section through the mask of Fig. 3L. The interior surface is also indicated.
Fig. 3O illustrates a left-hand rule.
Fig. 3P illustrates a right-hand rule.
Fig. 3Q shows a left ear, including the left ear helix.
Fig. 3R shows a right ear, including the right ear helix.
Fig. 3S shows a right-hand helix.
Fig. 3T shows a view of a mask, including the sign of the torsion of the space curve defined by the edge of the sealing membrane in different regions of the mask.
Fig. 3U shows a view of a plenum chamber 3200 showing a sagittal plane and a mid-contact plane.
Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

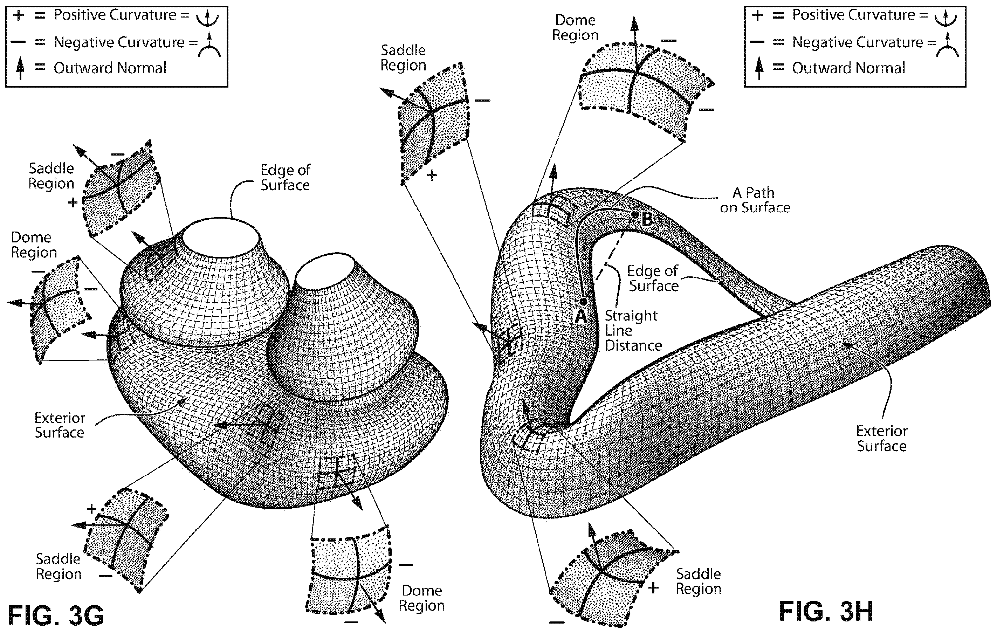

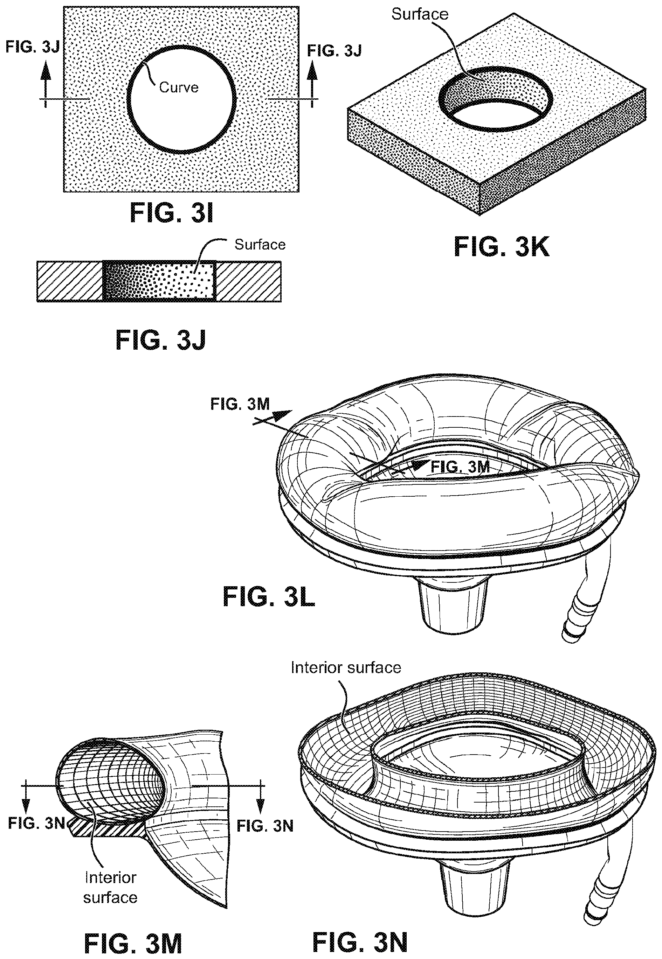

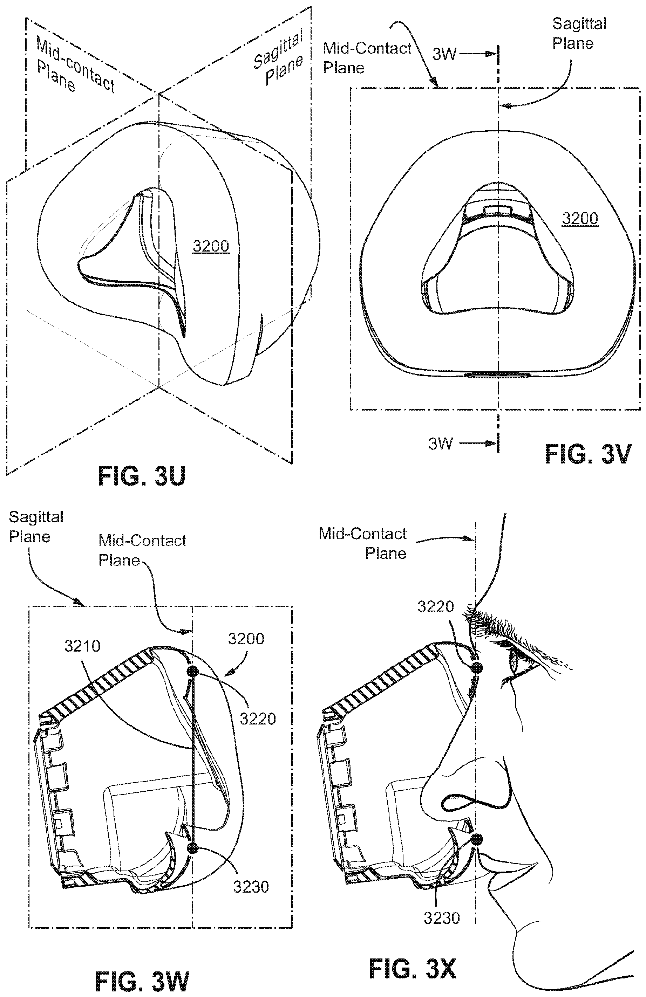

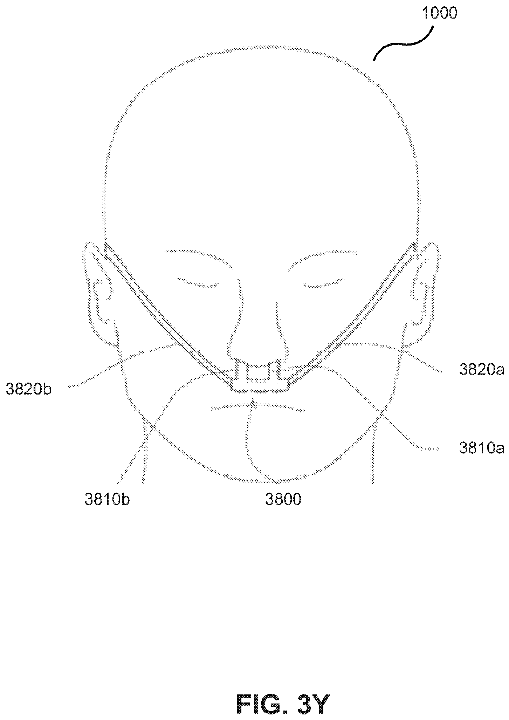

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.
Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.
Fig. 3Y shows a patient interface in the form of a nasal cannula in accordance with one form of the present technology.

### 3.4 RPT DEVICE

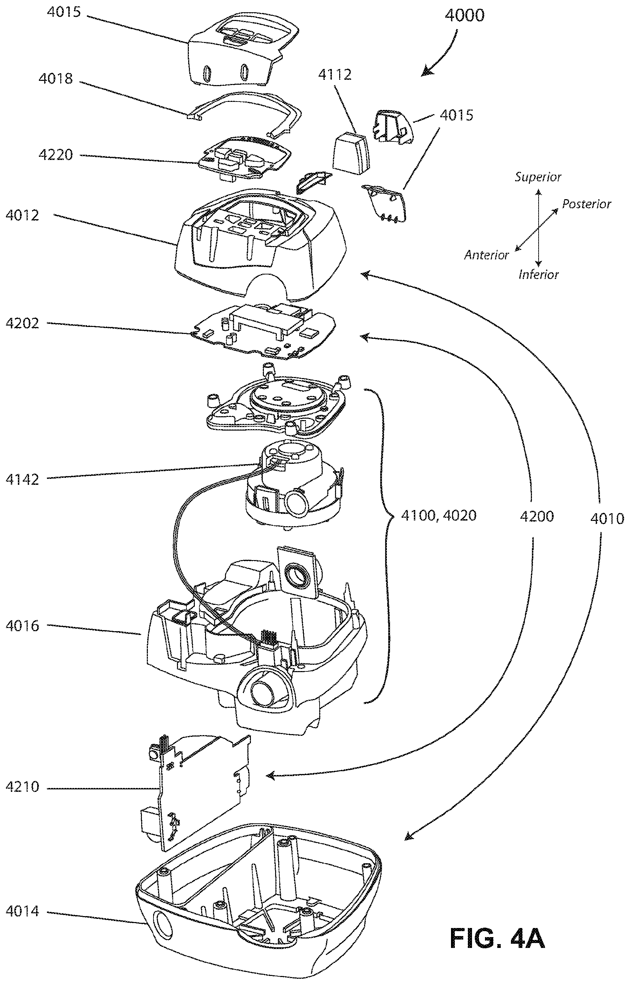

Fig. 4A shows an RPT device in accordance with one form of the present technology.

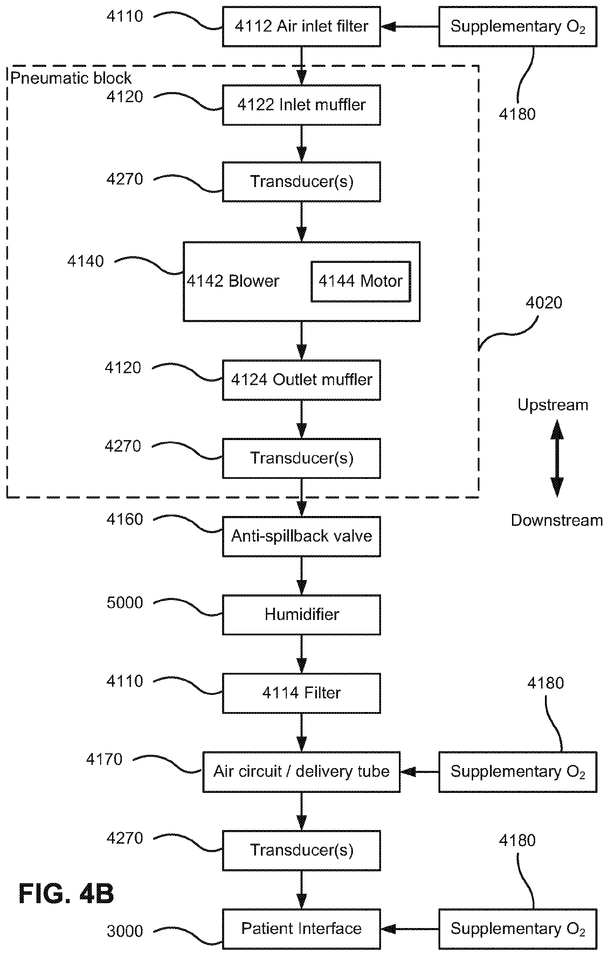

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 3.5 HUMIDIFIER

Fig. 5A shows an isometric view of a humidifier in accordance with one form of the present technology.
Fig. 5B shows an isometric view of a humidifier in accordance with one form of the present technology, showing a humidifier reservoir 5110 removed from the humidifier reservoir dock 5130.

### 3.6 EXAMPLES OF PATIENT CONTACTING MEMBERS OF THE PRESENT TECHNOLOGY

Fig. 6 shows a perspective view of a patient interface.
Fig. 7 is a perspective view illustration of a patient contacting member according to one example of the of the present technology engaged with a patient interface.
Fig. 8 is a perspective view illustration of a patient contacting member according to another example of the of the present technology engaged with a patient interface.
Fig. 9 is a perspective view illustration of a patient contacting member according to another example of the of the present technology engaged with a patient interface.
Fig. 9a is a perspective view illustration of a patient contacting member according to Fig. 9 with an alternative connector provided to the lower strap.
Fig. 10 is a perspective view illustration of a patient contacting member according to another example of the of the present technology engaged with a patient interface.
Fig. 11 is a side view illustration of a patient contacting member according to another example of the of the present technology engaged with a patient interface.
Fig. 12 is an enlarged side view of one form of a window portion of a patient contacting member according to another example of the of the present technology.
Fig. 13 is an enlarged side view of another form of a window portion of a patient contacting member according to another example of the of the present technology.
Fig. 14 is a perspective view illustration of a patient contacting member according to another example of the of the present technology.
Fig. 15 is a side view of a patient contacting member according to another example of the present technology, shown separated from a conduit of a positioning and stabilising structure.
Fig. 16 is a front view of the patient contacting member of Fig. 15 shown attached to a conduit of a positioning and stabilising structure.
Fig. 17 is a diagrammatic view of two patient contacting members having the same chord length but different arc lengths.
Fig. 18 shows a perspective view of a liner member according to one form of the technology.
Fig. 19 is a side view illustration of a gas delivery tube assembly according to one form of the technology comprising the liner member of Fig. 18 partially engaged with a conduit.
Fig. 20 is a side view of a patient contacting member according to one form of the technology, seen from the non-patient contacting side.
Fig. 21 is a side view of the patient contacting member of Fig. 20 seen from the patient contacting side.
Fig. 22 is an enlarged perspective view of the patient contacting member of Fig. 20 attached to a conduit of a positioning and stabilising structure.
Fig. 23 is a side view of the patient contacting member of Fig. 20 attached to a conduit of a positioning and stabilising structure.

### 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

### 4.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 4.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 or 3800.

### 4.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

An unsealed patient interface 3800, in the form of a nasal cannula, includes nasal prongs 3810a, 3810b which can deliver air to respective nares of the patient 1000 via respective orifices in their tips. Such nasal prongs do not generally form a seal with the inner or outer skin surface of the nares. The air to the nasal prongs may be delivered by one or more air supply lumens 3820a, 3820b that are coupled with the nasal cannula 3800. The lumens 3820a, 3820b lead from the nasal cannula 3800 to a respiratory therapy device via an air circuit. The unsealed patient interface 3800 is particularly suitable for delivery of flow therapies, in which the RPT device generates the flow of air at controlled flow rates rather than controlled pressures. The "vent" at the unsealed patient interface 3800, through which excess airflow escapes to ambient, is the passage between the end of the prongs 3810a and 3810b of the cannula 3800 via the patient's nares to atmosphere.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH₂O with respect to ambient.

The patient interface 3000 in accordance with one form of the present technology is constructed and arranged to be able to provide a supply of air at a positive pressure of at least 10 cmH₂O with respect to ambient.

The patient interface 3000 in accordance with one form of the present technology is constructed and arranged to be able to provide a supply of air at a positive pressure of at least 20 cmH₂O with respect to ambient.

### 4.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

### 4.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 4.3.1.2 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 4.3.2 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

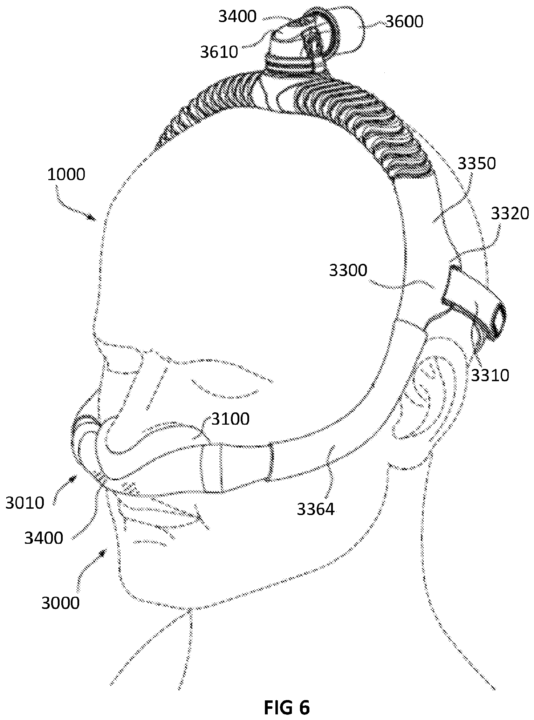

In some forms of the present technology, the positioning and stabilising structure 3300 comprises one or more tubes that deliver pressurised air received from a conduit forming part of the air circuit 4170 from the RPT device to the patient's airways, for example through an interfacing structure 3010 which comprises a plenum chamber 3200 and seal-forming structure 3100, for example as shown in Fig. 6. In examples, the positioning and stabilising structure 3300 comprises two tubes 3350 that deliver air to the seal-forming structure 3100 from the air circuit 4170. The tubes 3350 are an integral part of the positioning and stabilising structure 3300 of patient interface 3000 to position and stabilise the seal-forming structure 3100 of the patient interface to the appropriate part of the patient's face (for example, the nose and/or mouth). This allows the conduit of air circuit 4170 providing the flow of pressurised air to connect to a connection port 3600 of the patient interface in a position other than in front of the patient's face which may be unsightly to some people. While the use of a pair of tubes 3350 has some advantages (described below), in some examples the positioning and stabilising structure 3300 comprises only a single tube 3350 configured to overlie the patient's head on one side. A strap or other stabilising component may be provided to the other side of the patient's head between the top end of the single tube 3350 and the seal-forming structure 3100, to provide balanced forces on the seal-forming structure 3100.

In certain forms of the present technology, the patient interface 3000 may comprise a connection port 3600 located proximal a top, side or rear portion of a patient's head. For example, in the form of the present technology illustrated in Fig. 6 the connection port 3600 is located on top of the patient's head. In this example the patient interface 3000 comprises an elbow 3610 to which the connection port 3600 is provided. The elbow 3610 may swivel with respect to the positioning and stabilising structure 3300 in order to decouple movement of a conduit connected to the connection port 3600 from the positioning and stabilising structure 3300. The elbow 3610 may connect to a fluid connection opening in the headgear tubing 3350 or in a component to which the headgear tubing 3350 is connected. Additionally, or alternatively, a conduit connected to the connection port 3600 may swivel with respect to the elbow 3610. In the illustrated example, elbow 3610 comprises a swivelling conduit connector comprising the connection port 3600 to which a conduit of the air circuit 4170 is able to connect, such that the conduit can rotate about its longitudinal axis with respect to the elbow 3610. In some examples the air circuit 4170 may connect to the fluid connection opening. The elbow 3610 may rotatably connect to the fluid connection opening or to a ring received in the fluid connection opening.

In the example shown in Fig. 6, the two tubes 3350 are integrally formed and comprise a fluid connection opening to which the swivel elbow connects. In other examples, where separate tubes are used, they may be indirectly connected together, for example each may be connected to a T-shaped conduit having two conduit arms each fluidly connectable to the tubes 3350. The crown connector may comprise a third conduit arm. The connection port 3600 may comprise an elbow 3610 received at the centre of the crown connector 3360. The elbow 3610 may be configured to swivel.

### 4.3.3 Headgear strap connected to tubes

In certain forms of the present technology, for example as shown in Fig. 6, the positioning and stabilising structure 3300 comprises at least one headgear strap 3310 acting in addition to the tubes 3350 to position and stabilise the seal-forming structure 3100 in sealing position at the entrance to the patient's airways. In one example the patient interface 3000 comprises a strap 3310 forming part of the positioning and stabilising structure 3300. The strap 3310 may be known as a back strap or a rear headgear strap, for example. In other examples of the present technology, one or more further straps may be provided. For example, a patient interface 3000 according to an example of the present technology having a full face or oro-nasal cushion module may have a second, lower, strap configured to overlie the back of the patient's neck.

In certain examples of the present technology, the tubes 3350 are configured to receive a strap 3310 (for example by provision of tabs 3320) at the locations superior to and proximate the patient's ears. If the strap 3310 connects to the tubes 3350 too high with respect to the patient's head, the strap 3310 may have a tendency to ride up the back of the patient's head. Additionally, the strap 3310 could form too large an angle with respect to the superior portions of the headgear tubes 3350, resulting in the necessity for the patient to tighten the strap 3310 excessively, which could result in excessive tension in the positioning and stabilising structure 3300 and could make the strap 3310 more likely to ride up the back of the patient's head. Accordingly, it is advantageous for the connection between the strap 3310 and the tubes 3350 to be provided as low as possible, but spaced from the top of the patient's ear sufficiently that upon tightening of the strap 3310 the tubes 3350 are not pulled into contact with the patient's ears.

As is described below, in other forms of the technology the tubes 3350 are not configured to receive a strap 3310. In some forms of the technology the one or more straps may be connected to and/or may form part of a patient contacting member 3370, 3380. In these forms of the technology similar considerations to those discussed above apply in relation to the positioning of the strap(s).

### 4.3.4 Patient contacting members

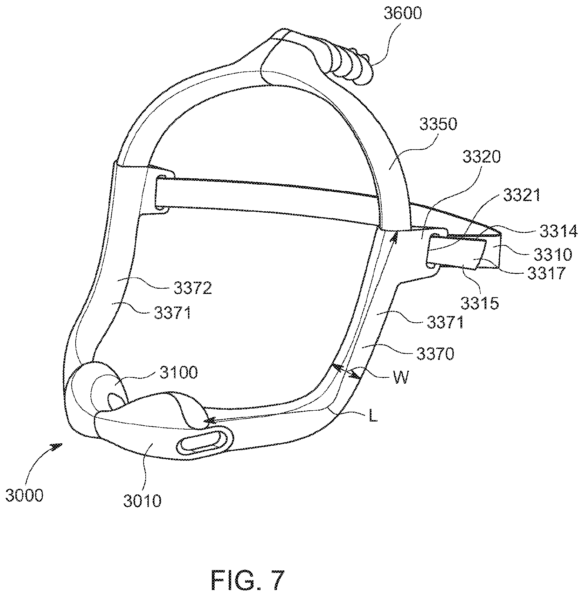

Referring next to Figs. 7, 8 and 9, in some forms of the technology a patient contacting member 3370 is provided. In the embodiments shown in Figs. 7 to 9 the patient contacting member 3370 comprises an elongate sleeve portion 3371 (or sleeve portion 3371) which is configured to releasably engage a tube 3350 (e.g. conduit) of the positioning and stabilising structure 3300. In one form of the technology, the elongate sleeve portion 3371 may be partially or completely formed from a textile. The textile may be woven or non-woven.

In examples the elongate sleeve portion 3371 may be made from an elastic material, or may comprise one or more portions made from an elastic material, to facilitate insertion of the tube 3350 through the elongate sleeve portion 3371. In examples the elongate sleeve portion 3371 may be dimensioned to allow the tube 3350 to slide through the elongate sleeve portion 3371, either with one or more elastic portions of the elongate sleeve portion 3371 stretched out, or without stretching out any portion of the elongate sleeve portion 3371. The sleeve portion(s) 3371 may be configured to allow repeated engagement and disengagement from the tube 3350 (e.g. without damage) by a user (e.g. a patient), rather than being engaged and/or manufactured with the tube 3350 by the manufacturer and being difficult or substantially impossible to disengage from the tube 3350 (at least without damaging the patient contacting member and/or the tube) by the patient. Fig. 6 shows a sleeve 3364 of the prior art which is of a type which is permanently engaged with the headgear tube 3350 by the manufacturer.

In some forms of the technology the patient contacting member 3370 further comprises a strap 3310, for example a back strap 3310 as described above.

In some forms of the technology, the patient contacting member 3370 comprises two sleeve portions 3371, each sleeve portion 3371 configured to connect to a respective tube 3350 provided on opposite sides of the positioning and stabilising structure 3300.

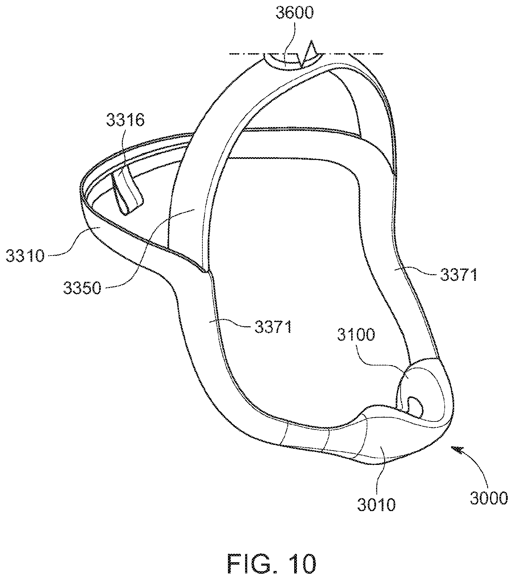

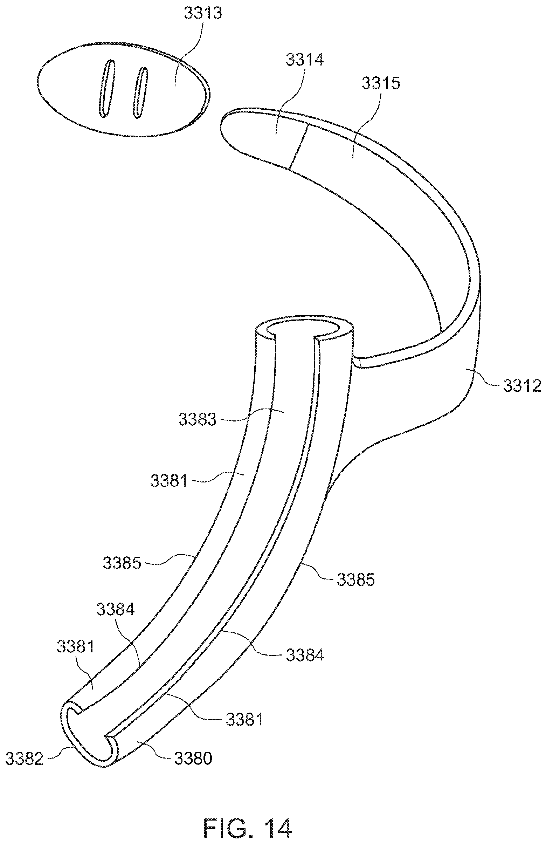

In the example shown in Fig. 10, two sleeve portions 3371 and a back strap 3310 are integrally formed as a single piece, for example from the same material. However, in other forms of the technology (for example as shown in Fig. 14) the back strap 3310 may comprise two separate back strap portions 3312 (only one of which is shown in Fig. 14) which are connectable together by a suitable connector means 3313, for example an adjustable connector means 3313 which allows the overall length of the back strap 3310 to be adjusted. In examples, a hook and loop fastening system (for example Velcro ^{™}) may be used. For example, the ends of the back strap portions 3312 may be provided with a portion of unbroken loop material 3314 and an adjacent portion of each back strap portion 3312 may be provided with a portion of broken loop (e.g. hook) material 3315, such that each back strap portion 3312 can be threaded through a suitable connector and can connect to itself. In other examples the positions of the broken loop material and unbroken loop material may be reversed on one or both of the back strap portions 3312. In other examples one of the back strap portions 3312 may be provided with a portion of broken loop material and the other back strap portion 3312 may be provided with a portion of unbroken loop material such that the two back strap portions 3312 can be connected together without the use of an intermediate connector means. Additionally or alternatively, one or more portions of the back strap 3310 may be formed from an elastically extensible material. In examples the back strap 3310 may be stretched over the patient's head when donning or doffing the interface, without the patient otherwise needing to adjust the length of the back strap 3310 or needing to disengage the back strap 3310 from the patient contacting member 3370.

As shown in Fig. 10, in one form of the technology the back strap 3310 may be provided with a tag 3316 which can be grasped by the patient to assist the patient in sliding the strap 3310 over their head and into position when donning the interface.

In the example shown in Fig. 7, the strap 3310 is releasably connectable to a strap engaging portion of the sleeve portions 3371. In this example the ends of the sleeve portions 3371 distal the interfacing structure 3010 are provided with loops or slots 3321 which can be engaged by the strap 3310. In the examples shown in Figs. 7, 8 and 9, each slot is provided in a tab 3320.

In one form of the technology one or both of the ends of the strap 3310 are provided with a portion of hook material 3315 which engages a portion of unbroken loop material 3314 provided on an adjacent portion of the strap 3310 to form an adjustable connection mechanism. In alternative forms of the technology the ends 3317 of the strap 3310 are provided with unbroken loop material 3314 and a hook material 3315 is provided to the exterior surface of the strap 3310 for engaging the unbroken loop material 3314. Other forms of connector are also possible, for example buckles, magnetic connectors or other connectors.

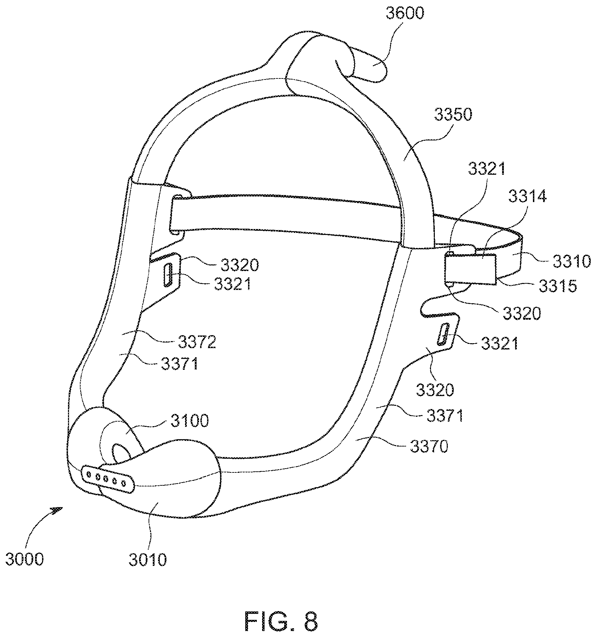

As shown in Fig. 8, in some forms of the technology each elongate sleeve portion 3371 comprises a plurality of strap engaging portions, e.g. loops, eyes or slots 3321. In examples the elongate sleeve portion 3371 may comprise a plurality of slots 3321, each of which can be selectively engaged by the strap 3310. This may allow the patient to choose the position of the strap 3310 relative to the elongate sleeve portion 3371 and/or the interfacing structure 3010. The loops, eyes or slots 3321 may be provided in tabs 3320 which may all be the same length, or may be two or more different lengths.

Selection of a particular strap engaging portion to connect the strap 3310 to may affect the balance of forces within the headgear, and may thereby affect the fit of the interface, as is described herein. This may allow a range of users of different sizes to comfortably use the same form and/or size of positioning and stabilising structure 3300. Tabs 3320 that extend further in the posterior direction may enable the back strap 3310 to connect at a more posterior location, which may reduce the tendency of the strap 3310 to ride up or down on the patient's head, resulting in a more stable positioning and stabilising structure 3300 for some users.

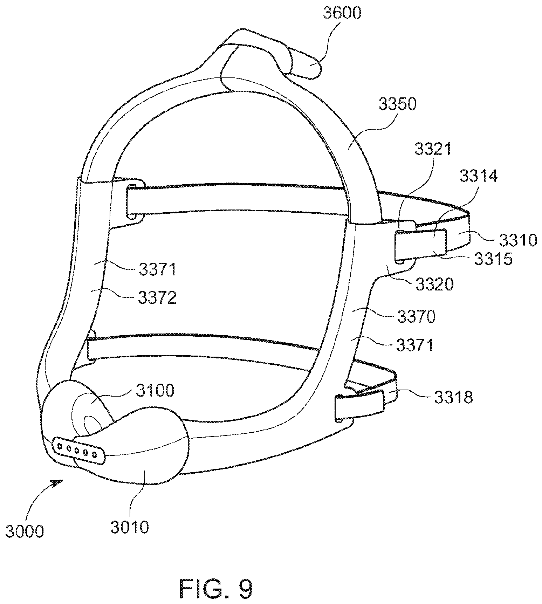

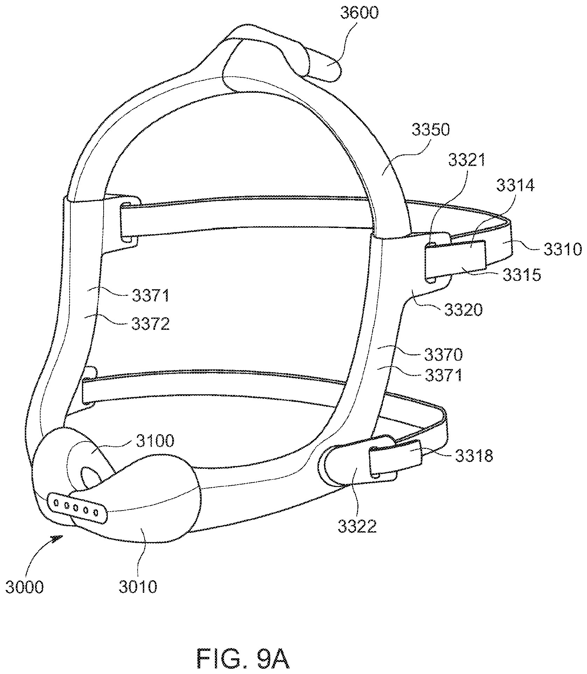

In one form of the technology, shown in Fig 9, the patient contacting member 3370 is provided with a further strap 3318 in addition to the back strap 3310 described above. In examples, the further strap 3318 is provided near or at an end of the elongate sleeve portion 3371 which is closest to, for example adjacent, the interfacing structure 3010, when in use (or is at least toward an inferior portion of the elongate sleeve portion). The further strap 3318 may extend around the patient's neck in use. This example may be particularly suitable for use with full face masks, ultra compact oro-nasal masks and/or nasal masks. In some examples the further strap 3318 may engage a slot in a tab 3320 via a hook and loop fastening system, as described above. In other examples the further strap 3318 may engage the tab 3320 via an alternative connector, for example a magnetic connector, as shown in Fig 9A.

In the examples shown in Figs. 7 to 9 and 9A, the elongate sleeve portion 3371 may have a patient contacting side 3372 which contacts at least the patient's cheek region in use.

Referring next to Fig. 7, in examples the length L along the elongate sleeve portion 3371 is greater than the width W of the elongate sleeve portion 3371. In some examples the length L (i.e. the length along the sleeve portion, e.g. the arc length), may be more than twice the width W of the elongate sleeve portion 3371 (the width being measured parallel to the surface of the patient's face), for example more than four times the width W. In one embodiment the elongate sleeve portion 3371 is dimensioned to cover substantially the entire portion of the patient contacting side 3351 of the tube 3350 between the interfacing structure 3010 and the back strap 3310. In other forms of the technology the elongate sleeve portion 3371 is dimensioned to cover the entire portion of the tube 3350 between the interfacing structure 3010 and the back strap 3310 (excluding any window portions provided, as discussed below). In some forms of the technology the patient contacting side 3372 of the elongate sleeve portion 3371 contacts the patient's face from a point immediately adjacent the interfacing structure 3010 to a point above the patient's ear.

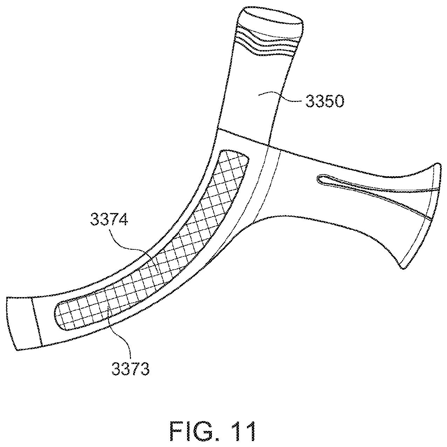

Referring next to Figs. 11 to 13, in some forms of the technology the non patient-contacting side of the patient contacting member 3370 (or sleeve 3370) may comprise a window portion 3373 which may be uncovered, covered by one or more fibres or cords which are sufficiently spaced apart to allow a user to see the tube 3350 through the window portion 3373, and/or covered with a transparent material. This may allow the user to check that the tube 3350 is clean (for example when the tube is itself transparent or translucent). Additionally or alternatively, the window portion 3373 may be configured to increase the flexibility of the sleeve 3370, thereby making the sleeve 3370 easier to engage with or disengage from the tube 3350 without damage.

In the example shown in Fig. 11 the window portion 3373 comprises an open mesh panel 3374. In one form of the technology the mesh panel 3374 may be knitted to shape (e.g. integrally formed) with the sleeve.

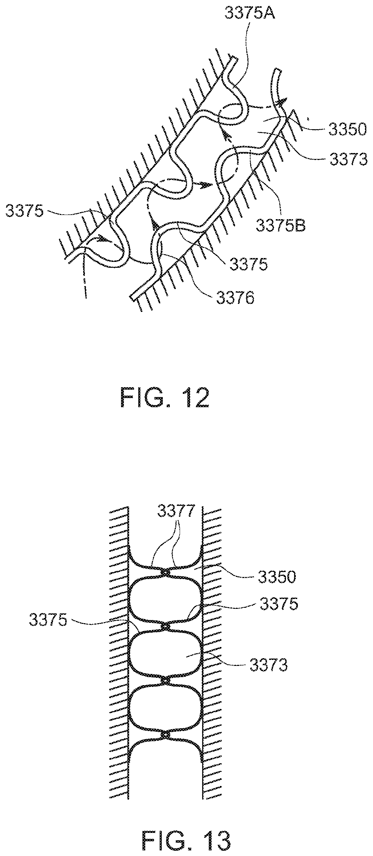

In another form of the technology, shown in Fig. 12, opposing sides of the window portion 3373 may comprise spaced apart loops 3375. The loops 3375A provided to one side of the window portion 3373 may be offset along the length of the window portion 3373 relative to the loops 3375B on the opposite side of the window portion 3373. The opposing loops 3375A, 3375B may be interconnected by one or more suitable fibres or cords 3376.

In another form of the technology, shown in Fig. 13, interlinked loops 3375 of shock cord 3377 may be provided to opposite sides of the window portion 3373. In the example shown the loops 3375 are provided in interlinked pairs, with one loop in the pair opposite (e.g. directly opposite) the other loop of the pair. The shock cord 3377 may be elastically extendible in length, meaning the circumference of the elongate sleeve portion 3371 is elastically enlargeable in that region, in the sense that the circumference can be increased by exerting a suitable force, but is biased towards an original circumference by the interlinked shock cords 3377.

In other forms of the technology the window portion 3373 may comprise a transparent material, for example a silicone panel.

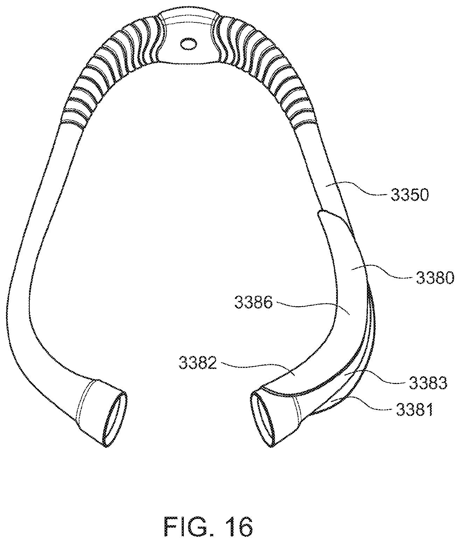

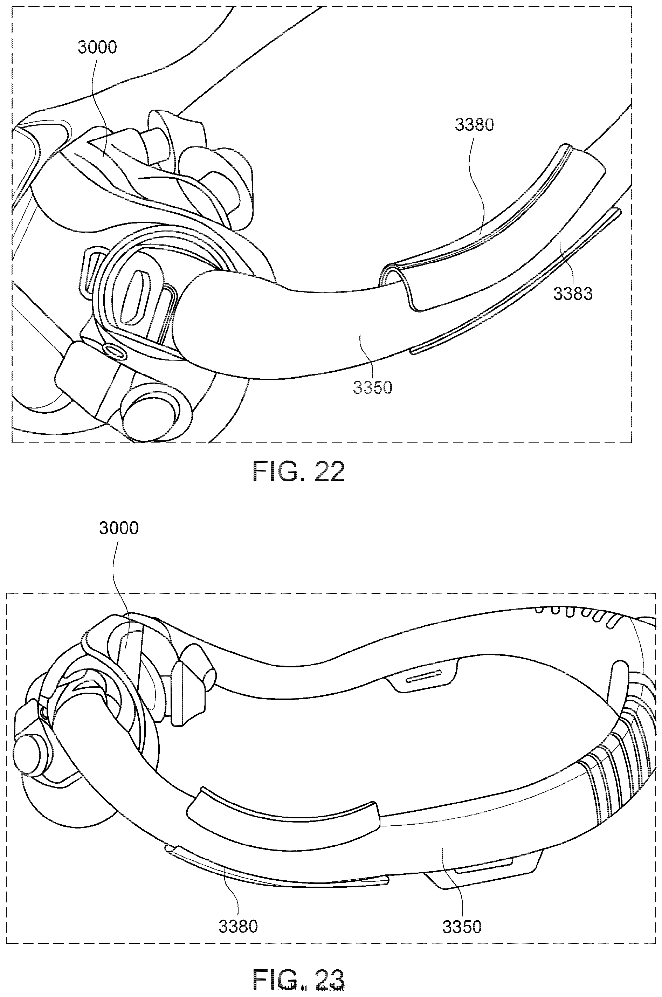

In other forms of the technology, shown in Figs. 14 to 16 and 20 to 23, a patient contacting member 3380 comprises a body 3381 configured to at least partially wrap around a tube 3350 of a positioning and stabilising structure 3300, wherein the body 3381 comprises a patient contacting side 3382 and slot 3383 on an opposite side of the body 3381 to the patent contacting side.

The slot 3383 may extend substantially longitudinally along the patient contacting member 3380, that is, along the entire length of the patient contacting member 3380. In some forms of the technology the slot 3383 may be curved, for example, if the body 3381 is curved, the sides 3384 of the slot 3383 may also be curved so as to be substantially parallel to the sides 3385 of the body 3381. In other forms of the technology the sides 3384 of the slot 3383 may be non-parallel to each other and/or to the sides 3385 of the body 3381.

In some forms of the technology the body 3381, or at least a portion of the body 3381, may be rigidised, that is, may have a greater resistance to bending than a tube 3350 which it is intended to engage, in use. The slot 3383 may be shaped and dimensioned such that the patient contacting member 3380 can be engaged over a tube 3350 of a positioning and stabilising structure 3300 by inserting the tube 3350 through the slot 3383 (or considered another way, by snapping the body 3381 over the tube). In some forms of the technology, portions of the body 3381 adjacent the slot 3383 and/or which form the edges of the slot 3383 are resiliently flexible to form a clipping portion which makes engagement of the patient contacting member 3380 over the tube 3350 easier. In other forms of the technology the body 3381 may be substantially rigid and the tube 3350 may be sufficiently deformable to allow insertion through the slot 3383. In some forms of the technology the sides 3384 of the slot 3383 may be generally parallel to each other, but one or more portions may of the sides 3384 may be closer together (e.g. to reduce the width of the slot 3383). These portions may ensure that the body 3381 engages the tube 3350 securely.

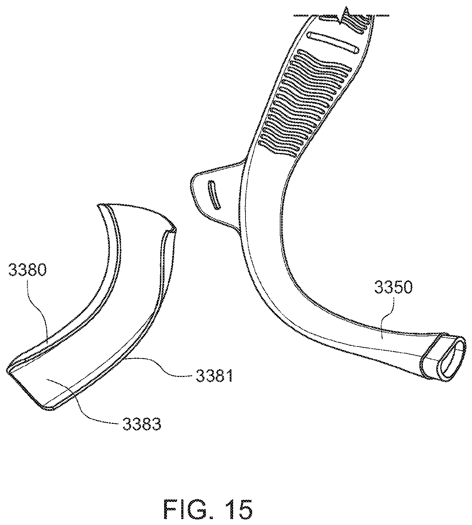

In examples the body 3381 may be formed from a textile laminate. The laminate may comprise a textile and one or more of a silicone, foam or other plastic material, for example with the textile layer 3386 provided as an outer layer of the laminate so as to be in contact with the patient's skin. In examples the body 3381 may be thermoformed to provide rigidity to selected areas. In embodiments with integral straps 3310, for example as shown in Fig. 14, the strap portion 3312 may not be thermoformed, thereby maintaining its flexibility. Alternatively, a strap portion 3312 may be formed from a separate piece of material. As shown in Figs. 15, 16 and 20 to 23, some embodiments of the patient contacting member 3380 may not have a strap 3310 or a strap engaging portion.

In some forms of the technology the opposed edges of the slot 3383 may touch along some or all of their length, or one edge may overlap the other along some or all of its length, when the patient contacting member 3380 has been engaged with a tube 3350 of a positioning and stabilising structure 3300. For example, the portions of the body 3381 adjacent the slot 3383 and/or which form the edges of the slot 3383 may be flexible enough to allow a user to move the edges of the slot apart to allow the tube 3350 to be inserted into the patient contacting member 3380, and may then return to a configuration in which the edges are in contact, or in which one edge overlaps the other.

In another form of the technology the edges of the slot 3383 may not contact each other or overlap, but an additional portion of more flexible material (for example a flap) may be provided to one or both edges 3383 to cover the slot when the patient contacting member 3380 is engaged with the tube 3350. The portion of material or flap may be connectable to the opposite edge of the slot (or to the material attached to the other side of the slot) by a suitable fastener, for example a hook and loop fastening system.

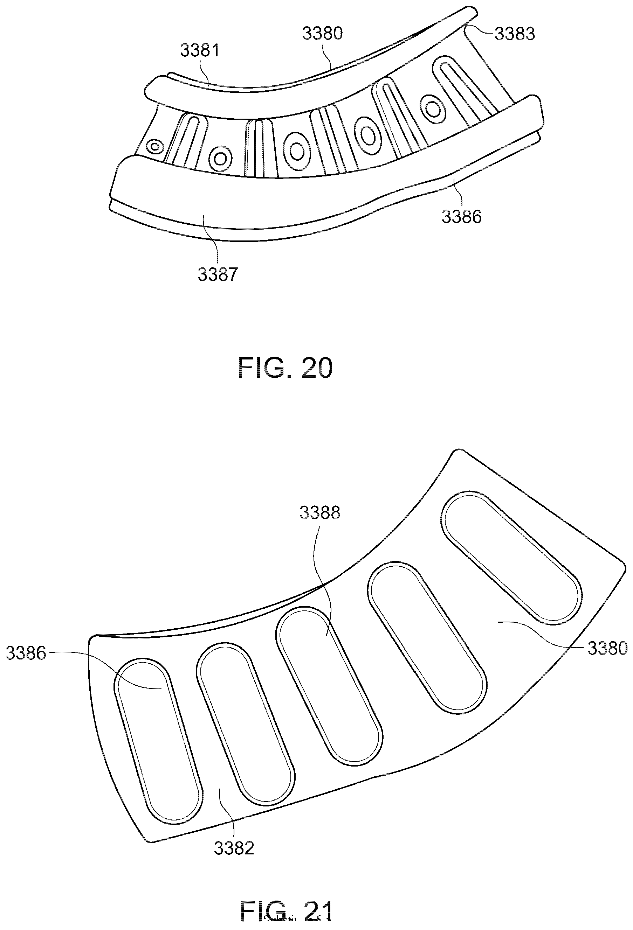

In the example shown in Figs. 20 to 23, the patient contacting member 3380 may comprise a body 3381 formed from a textile layer 3386 connected to a thermoplastic elastomer 3387, for example by overmoulding. The patient contacting side 3382 of the body 3381 may be provided with a plurality of protruding rib formations 3388.

The protruding rib formations 3388 may provide regions between the rib formations in which there is no contact between the patient's skin and the patient contacting side 3382 of the body 3381. These regions may allow the patient's skin to breath.

Patient contacting members 3380 featuring such rib formations 3388 may also be relatively flexible in the longitudinal direction, while maintaining a required stiffness in the transverse direction.

In other examples, patient contacting members 3380 comprising a textile layer 3386 connected to a thermoplastic elastomer 3387 (e.g by overmoulding) may be formed without rib formations 3388 and may have a substantially flat patient contacting side 3382.

### 4.3.5 System for positioning and stabilising

In some forms of the technology a system for positioning and stabilising a patient interfacing portion may comprise a positioning and stabilising structure 3300 comprising at least one gas delivery tube 3350, and a plurality of patient contacting members 3370, 3380 as described above. Each of the patient contacting members 3370, 3380 may be interchangeably engageable with the gas delivery tube 3350, that is, each patient contacting member can be engaged with the gas delivery tube 3350 if the others have first been removed.

In one form of the technology at least one of the patient contacting members 3370, 3380 may comprise a rigidised portion, or may be entirely rigidised, that is, it may have a greater resistance to bending in at least one plane (e.g. parallel to the surface of the patient's cheek) than the corresponding tube 3350.

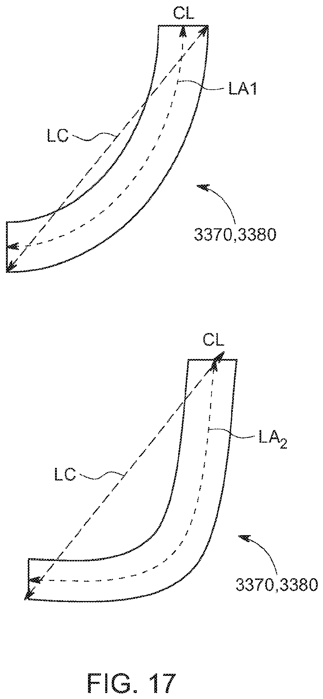

One or more of the partially or entirely rigidised patient contacting members 3370, 3380 may be curved along a portion of, or the entirety of, its longest dimension (i.e. length). As shown in Fig. 17, the patient contact member 3370, 3380 may have a chord length LC (e.g. straight line distance) and an arc length LA, where the arc length LA is the distance along the at least partially curved centreline CL of the patient contacting member. As seen in Fig. 17, patient contacting members 3370, 3380 having different curvatures may have different arc lengths LA₁, LA₂, but the same chord length LC. The less curved the patient contacting member is, the closer the arc length LA and the chord length LC will be.

Engaging such a rigidized patient contacting member 3370, 3380 with a tube 3350 of the positioning and stabilising structure 3300 may result in the portion of the tube 3350 which is in contact with patient contact member substantially adopting the curvature of the patient contacting member 3370, 3380. Therefore, in one form of the technology a positioning and stabilising structure 3300 comprising a tube 3350 may be adjusted to fit a particular patient by engaging a patient contacting member 3370, 3380 having a suitable arc length LA and chord length LC. Because a plurality of patient contacting members 3370, 3380 can have the same chord length but different arc lengths, and a plurality of patient contacting members 3370, 3380 can have the same arc length but different chord lengths, a patient can select a patient contacting member 3370, 3380 which both adjusts the fit of the positioning and stabilising structure 3300 to the particular patient's head, and which locates a strap engaging portion and/or an integral back strap 3310 in a suitable position on the patient's head. In this way a single size of positioning and stabilising structure 3300 may be adapted for use by patients with a larger range of head shapes and sizes than similar positioning and stabilising structures of the prior art.

In one form of the technology a system for positioning and stabilising a patient interface portion may comprise a positioning and stabilising structure 3300 comprising at least one gas delivery tube 3350 and a plurality of patient contacting members 3370, 3380 as described above, where a plurality of the patient contacting members 3370, 3380 have the same chord length but different arc lengths. In another form of the technology a system for positioning and stabilising a patient interface may comprise a positioning and stabilising structure 3300 comprising at least one gas delivery tube 3350 and a plurality of patient contacting members 3370, 3380 as described above, where a plurality of the patient contacting members have the same arc length but different chord lengths. In another form of the technology a system for positioning and stabilising a patient interface may comprise a positioning and stabilising structure 3300 comprising at least one gas delivery tube 3350 and a plurality of patient contacting members 3370, 3380 as described above, where a first group of the patient contacting members has the same arc length but different chord lengths and a second group of the patient contacting members has the same chord length but different arc lengths, wherein the first and second groups each comprise a plurality of patient contacting members.

### 4.3.6 Gas delivery tube assembly

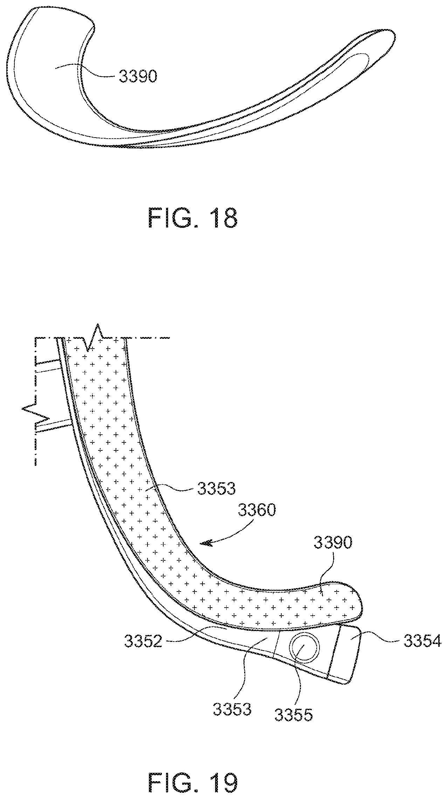

Referring next to Figs. 18 and 19, in another form of the technology a gas delivery tube assembly 3360 for use with a patient interface, for example a gas delivery tube assembly 3360 which forms part of a positioning and stabilising structure 3300 for the patient interface, comprises a gas delivery tube 3352 which is releasably connectable to a liner member 3390.

The gas delivery tube 3352 has a patient-facing side 3353 and a non patient-facing side 3354. In some examples the patient-facing side 3353 may contact the patient, although in examples some or all of the patient-facing side 3353 of the gas delivery tube may not be in contact with the patient due to the presence of the liner member 3390.

At least one of the patient-facing side 3353 of the gas delivery tube 3352 and the liner member 3390 may comprise at least one section of unbroken loop material, and the other of the patient facing side 3353 of the gas delivery tube 3352 and the liner member 3390 comprises at least one section of hook or broken loop material configured to releasably engage the unbroken loop material. In the example shown in Fig. 19, the gas delivery tube 3352 is provided with a plurality of portions of broken loop material 3355 spaced apart along the length of the tube 3352. The liner member 3390 may comprise a plurality of portions of unbroken loop material, or may comprise a single portion of unbroken loop material along substantially an entire length of the liner member 3390.

The liner member 3390 may comprise a laminate, for example comprising a textile outer (patient contacting) layer, and at least one cushioning layer, for example a layer of foam. In some forms of the technology the laminate may be sufficiently thick that portions of the patient facing side 3353 of the gas delivery tube immediately adjacent the ends of the liner member 3390 are held away from the patient's skin in use. In one form of the technology the liner is at least substantially 2mm thick, for example 2mm-10mm, for example 4mm-5mm.

The section(s) of unbroken loop material and/or broken loop material provided to the gas delivery tube 3352 may be moulded into the gas delivery tube 3352 or may be connected by an adhesive or other suitable joining method.

In examples the gas delivery tube assembly 3360 described above may be used in combination with a patient contacting member 3370, 3380 as described above.

### 4.3.7 Vent

In one form, the patient interface 3000 includes a vent 3400 constructed and arranged to allow for the washout of exhaled gases, e.g. carbon dioxide.

### 4.3.8 Decoupling structure(s)

In one form the patient interface 3000 includes at least one decoupling structure, for example, a swivel or a ball and socket.

### 4.3.9 Connection port

Connection port 3600 allows for connection to the air circuit 4170.

### 4.3.10 Forehead support

In one form, the patient interface 3000 includes a forehead support 3700.

### 4.3.11 Anti-asphyxia valve

In one form, the patient interface 3000 includes an anti-asphyxia valve.

### 4.3.12 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 4.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH₂O, or at least 10cmH₂O, or at least 20 cmH₂O.

### 4.5 HUMIDIFIER

### 4.5.1 Humidifier overview

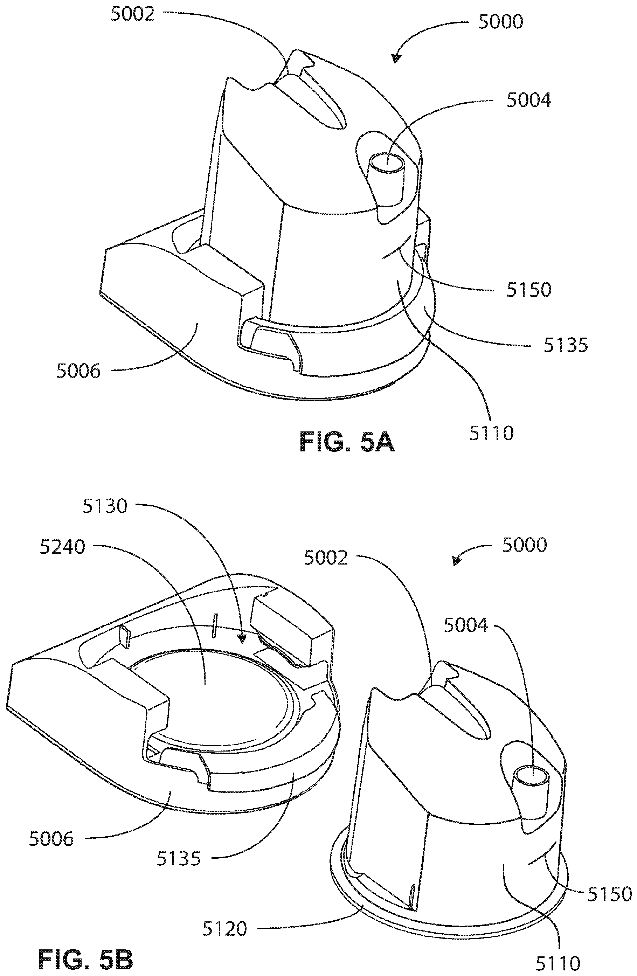

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of air. In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 4.6 RESPIRATORY THERAPY MODES

Various respiratory therapy modes may be implemented by the disclosed respiratory therapy system.

### 4.7 GLOSSARY

For the purposes of the present technology disclosure, in certain forms of the present technology, one or more of the following definitions may apply. In other forms of the present technology, alternative definitions may apply.

### 4.7.1 General

*Air:* In certain forms of the present technology, air may be taken to mean atmospheric air, and in other forms of the present technology air may be taken to mean some other combination of breathable gases, e.g. atmospheric air enriched with oxygen.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy (RPT):* The application of a supply of air to an entrance to the airways at a treatment pressure that is typically positive with respect to atmosphere.

*Ventilator:* A mechanical device that provides pressure support to a patient to perform some or all of the work of breathing.

### 4.7.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 4.7.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 4.7.2 Anatomy

### 4.7.2.1 Anatomy of the face

*Ala:* the external outer wall or "wing" of each nostril (plural: alar)

*Alare:* The most lateral point on the nasal *ala.*

*Alar curvature (or alar crest) point:* The most posterior point in the curved base line of each *ala*, found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework:* The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane:* A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

*Greater alar cartilage:* A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils):* Approximately ellipsoidal apertures forming the entrance to the nasal cavity. The singular form of nares is naris (nostril). The nares are separated by the nasal septum.

*Naso-labial sulcus or Naso-labial fold:* The skin fold or groove that runs from each side of the nose to the corners of the mouth, separating the cheeks from the upper lip.

*Naso-labial angle:* The angle between the columella and the upper lip, while intersecting subnasale.

*Otobasion inferior:* The lowest point of attachment of the auricle to the skin of the face.

*Otobasion superior:* The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal):* The nasal ridge is the midline prominence of the nose, extending from the Sellion to the Pronasale.

*Sagittal plane:* A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare*: The point at the lower margin of the alar base, where the alar base joins with the skin of the superior (upper) lip.

*Subnasal point:* Located on the soft tissue, the point at which the columella merges with the upper lip in the midsagittal plane.

*Supramenton:* The point of greatest concavity in the midline of the lower lip between labrale inferius and soft tissue pogonion

### 4.7.2.2 Anatomy of the skull

*Frontal bone:* The frontal bone includes a large vertical portion, the *squama frontalis,* corresponding to the region known as the forehead.

*Mandible:* The mandible forms the lower jaw. The mental protuberance is the bony protuberance of the jaw that forms the chin.

*Maxilla:* The maxilla forms the upper jaw and is located above the mandible and below the orbits. The *frontal process of the maxilla* projects upwards by the side of the nose, and forms part of its lateral boundary.

*Nasal bones:* The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone:* The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit:* The bony cavity in the skull to contain the eyeball.

*Parietal bones:* The parietal bones are the bones that, when joined together, form the roof and sides of the cranium.

*Temporal bones:* The temporal bones are situated on the bases and sides of the skull, and support that part of the face known as the temple.

*Zygomatic bones:* The face includes two zygomatic bones, located in the upper and lateral parts of the face and forming the prominence of the cheek.

### 4.7.2.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 4.7.3 Patient interface

*Anti-asphyxia valve (AAV):* The component or sub-assembly of a mask system that, by opening to atmosphere in a failsafe manner, reduces the risk of excessive CO₂ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 4.7.4 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

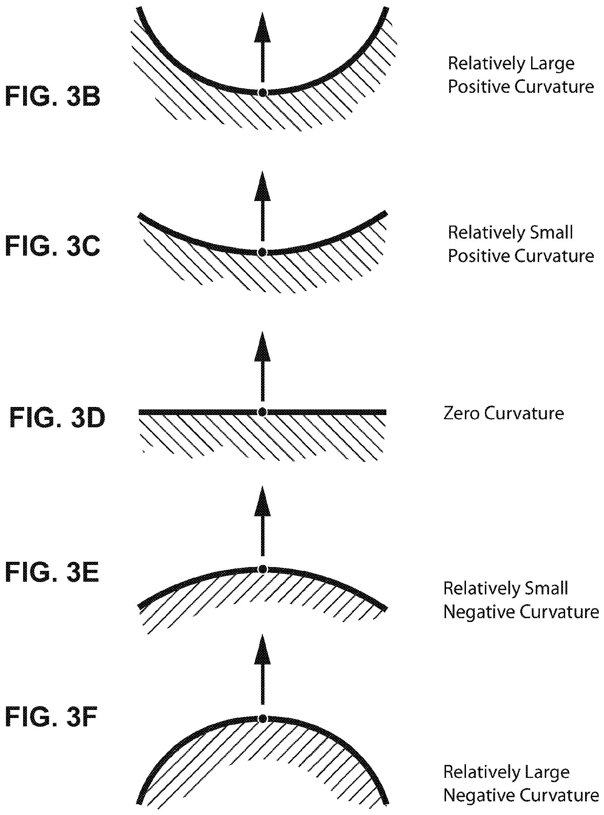

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 4.7.4.1 Curvature in one dimension

The curvature of a plane curve at *p* may be described as having a sign (e.g. positive, negative) and a magnitude (e.g. 1/radius of a circle that just touches the curve at *p*).

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 4.7.4.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from *f*(0) to *f*(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 4.7.4.3 Space curves

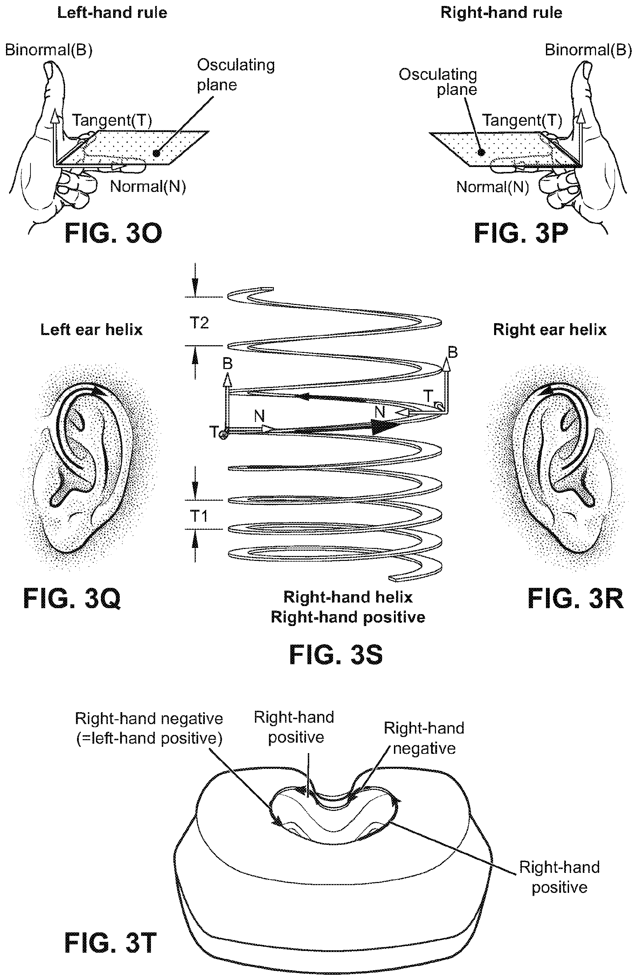

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

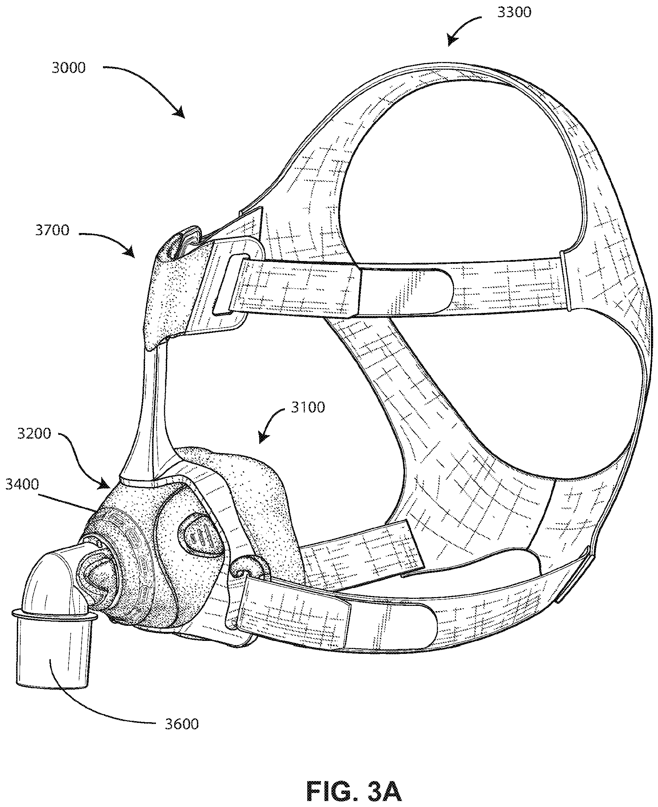

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 4.7.4.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 4.8 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology

### 4.9 REFERENCE SIGNS LIST

| | |
|---|---|
| patient | 1000 |
| bed partner | 1100 |
| patient interface | 3000 |
| interfacing structure | 3010 |
| seal forming structure | 3100 |
| plenum chamber | 3200 |
| chord | 3210 |
| superior point | 3220 |
| inferior point | 3230 |
| positioning and stabilising structure | 3300 |
| strap | 3310 |
| back strap portion | 3312 |
| connector | 3313 |
| unbroken loop | 3314 |
| broken loop | 3315 |
| tag | 3316 |
| ends | 3317 |
| further strap | 3318 |
| tab | 3320 |
| loop or slot | 3321 |
| magnetic connector | 3322 |
| tubes | 3350 |
| patient contacting side | 3351 |
| tube | 3352 |
| patient facing side | 3353 |
| non patient facing side | 3354 |
| broken loop | 3355 |
| tube assembly | 3360 |
| prior art sleeve | 3364 |
| patient contacting member | 3370 |
| elongate sleeve portion | 3371 |
| patient contacting side | 3372 |
| window | 3373 |
| mesh panel | 3374 |
| loops | 3375 |
| loops | 3375A |
| loop | 3375B |
| cord or fibre | 3376 |
| shock cord | 3377 |
| patient contacting member | 3380 |
| body | 3381 |
| patient contacting side | 3382 |
| slot | 3383 |
| sides of slot | 3384 |
| sides of body | 3385 |
| textile layer | 3386 |
| Elastomer | 3387 |
| rib formation | 3388 |
| liner | 3390 |
| vent | 3400 |
| connection port | 3600 |
| elbow | 3610 |
| forehead support | 3700 |
| patient interface | 3800 |
| RPT device | 4000 |
| external housing | 4010 |
| upper portion | 4012 |
| lower portion | 4014 |
| panel | 4015 |
| chassis | 4016 |
| handle | 4018 |
| pneumatic block | 4020 |
| assembly | 4100 |
| air filter | 4110 |
| inlet air filter | 4112 |
| filter | 4114 |
| muffler | 4120 |
| Inlet muffler | 4122 |
| outlet muffler | 4124 |
| pressure generator | 4140 |
| blower | 4142 |
| motor | 4144 |
| anti-spillback valve | 4160 |
| air circuit | 4170 |
| supplementary gas | 4180 |
| electrical components | 4200 |
| PCBA | 4202 |
| electrical power supply | 4210 |
| input device | 4220 |
| transducers | 4270 |
| humidifier | 5000 |
| humidifier inlet | 5002 |
| humidifier outlet | 5004 |
| humidifier base | 5006 |
| humidifier reservoir | 5110 |
| conductive portion | 5120 |
| reservoir dock | 5130 |
| locking lever | 5135 |
| water level indicator | 5150 |
| heating element | 5240 |

## Claims

1. A patient contacting member (3370) configured to releasably engage a gas delivery tube (3350) which forms part of a positioning and stabilising structure (3300) for a patient interface (3000), the patient contacting member (3370) comprising an elongate sleeve portion (3371) which is engageable with the gas delivery tube (3350) and a strap (3310) configured to engage a back of a patient's head, in use,
the elongate sleeve portion (3371) comprising a patient contacting side (3372) and a non-patient contacting side, wherein the patient contacting side (3372) contacts at least the patient's cheek region, in use,
**characterised in that** the non-patient contacting side comprises a window portion (3373),
wherein the window portion (3373) comprises a cover which allows the patient to see through the window portion (3373).

2. The patient contacting member (3370) of claim 1, wherein the strap (3310) is releasably connectable to the elongate sleeve portion (3371), and the elongate sleeve portion (3371) comprises a receiving means comprising an opening for receiving the strap (3310).

3. The patient contacting member (3370) of claim 2, wherein the elongate sleeve portion (3371) comprises a plurality of receiving means spaced apart along the elongate sleeve portion (3371).

4. The patient contacting member (3370) of claim 2 or 3, wherein the or each receiving means comprises a tab (3320) provided with a slot (3321) to receive the strap (3310).

5. The patient contacting member (3370) of any one of claims 2 to 4, wherein the patient contacting member (3370) comprises a further strap or a strap portion (3318), the further strap or strap portion (3318) configured to pass around the back of the patient's neck, in use.

6. The patient contacting member (3370) of any one of claims 1 to 5, wherein the elongate sleeve portion (3371) comprises a rigidized portion.

7. The patient contacting member (3370) of claim 6, wherein the rigidized portion is curved.

8. The patient contacting member (3370) of any one of claims 1 to 7, wherein the window portion (3373) comprises a first side and an opposite second side, and wherein the cover comprises an elasticated cord (3376, 3377) which is engaged with the first side and the second side.

9. The patient contacting member (3370) of claim 8, wherein the first side is provided with a plurality of first loops (3375, 3375A) and the second side is provided with a plurality of second loops (3375, 3375B), wherein the elasticated cord (3376, 3377) extends through a plurality of the first loops (3375, 3375A) and a plurality of the second loops (3375, 3375B).

10. The patient contacting member (3370) of any one of claims 1 to 7, wherein the cover comprises a mesh (3374).

11. The patient contacting member (3370) of any one of claims 1 to 7, wherein the cover comprises a transparent material.

## Patentansprüche

1. Patientenkontaktelement (3370), das zum lösbaren Eingriff mit einem Gaszuführschlauch (3350) ausgebildet ist, der Teil einer Positionierungs- und Stabilisierungsstruktur (3300) für eine Patientenschnittstelle (3000) ist, wobei das Patientenkontaktelement (3370) einen länglichen Hülsenabschnitt (3371), der mit dem Gaszuführschlauch (3350) in Eingriff gebracht werden kann, und ein Band (3310) aufweist, das dazu ausgebildet ist, im Gebrauch einen Hinterkopf eines Patienten in Eingriff zu nehmen,
wobei der längliche Hülsenabschnitt (3371) eine den Patienten berührende Seite (3372) und eine den Patienten nicht berührende Seite aufweist, wobei die den Patienten berührende Seite (3372) im Gebrauch mindestens den Wangenbereich des Patienten berührt,
**dadurch gekennzeichnet, dass**
die den Patienten nicht berührende Seite einen Fensterabschnitt (3373) aufweist,
wobei der Fensterabschnitt (3373) eine Abdeckung aufweist, die es dem Patienten ermöglicht, durch den Fensterabschnitt (3373) zu sehen.

2. Patientenkontaktelement (3370) nach Anspruch 1, wobei das Band (3310) lösbar mit dem länglichen Hülsenabschnitt (3371) verbunden werden kann und der längliche Hülsenabschnitt (3371) ein Aufnahmemittel mit einer Öffnung zum Aufnehmen des Bandes (3310) aufweist.

3. Patientenkontaktelement (3370) nach Anspruch 2, wobei der längliche Hülsenabschnitt (3371) mehrere Aufnahmemittel aufweist, die entlang des länglichen Hülsenabschnitts (3371) beabstandet sind.

4. Patientenkontaktelement (3370) nach Anspruch 2 oder 3, wobei das oder jedes Aufnahmemittel eine Lasche (3320) aufweist, die mit einem Schlitz (3321) zum Aufnehmen des Bandes (3310) versehen ist.

5. Patientenkontaktelement (3370) nach einem der Ansprüche 2 bis 4,
wobei das Patientenkontaktelement (3370) ein weiteres Band oder einen Bandabschnitt (3318) aufweist, wobei das weitere Band oder der Bandabschnitt (3318) dazu ausgebildet ist, im Gebrauch um den Nacken des Patienten herum zu verlaufen.

6. Patientenkontaktelement (3370) nach einem der Ansprüche 1 bis 5, wobei der längliche Hülsenabschnitt (3371) einen versteiften Abschnitt aufweist.

7. Patientenkontaktelement (3370) nach Anspruch 6, wobei der versteifte Abschnitt gekrümmt ist.

8. Patientenkontaktelement (3370) nach einem der Ansprüche 1 bis 7, wobei der Fensterabschnitt (3373) eine erste Seite und eine gegenüberliegende zweite Seite aufweist, und wobei die Abdeckung eine elastische Schnur (3376, 3377) aufweist, die sich mit der ersten Seite und der zweiten Seite in Eingriff befindet.

9. Patientenkontaktelement (3370) nach Anspruch 8, wobei die erste Seite mit mehreren ersten Schlaufen (3375, 3375A) und die zweite Seite mit mehreren zweiten Schlaufen (3375, 3375B) versehen ist, wobei sich die elastische Schnur (3376, 3377) durch mehrere der ersten Schlaufen (3375, 3375A) und mehrere der zweiten Schlaufen (3375, 3375B) erstreckt.

10. Patientenkontaktelement (3370) nach einem der Ansprüche 1 bis 7, wobei die Abdeckung ein Netz (3374) aufweist.

11. Patientenkontaktelement (3370) nach einem der Ansprüche 1 bis 7, wobei die Abdeckung ein transparentes Material aufweist.

## Revendications

1. Élément de contact avec un patient (3370) configuré pour raccorder de manière libérable un tube d'administration de gaz (3350) qui fait partie d'une structure de positionnement et de stabilisation (3300) d'une interface patient (3000), l'élément de contact avec un patient (3370) comprenant une partie manchon allongée (3371) qui peut être raccordée au tube d'administration de gaz (3350) et une sangle (3310) configurée pour coopérer avec l'arrière de la tête d'un patient, en utilisation,
la partie manchon allongée (3371) comprenant un côté en contact avec le patient (3372) et un côté sans contact avec le patient, dans lequel le côté en contact avec le patient (3372) contacte au moins la région de joue du patient, en utilisation,
**caractérisé en ce que**
le côté sans contact avec le patient comprend une partie fenêtre (3373),
dans lequel la partie fenêtre (3373) comprend un élément de recouvrement qui permet au patient de voir à travers la partie fenêtre (3373).

2. Élément de contact avec un patient (3370) selon la revendication 1, dans lequel la sangle (3310) peut être connectée de manière libérable à la partie manchon allongée (3371), et la partie manchon allongée (3371) comprend un moyen de réception comprenant une ouverture destinée à recevoir la sangle (3310).

3. Élément de contact avec un patient (3370) selon la revendication 2, dans lequel la partie manchon allongée (3371) comprend une pluralité de moyens de réception espacés le long de la partie manchon allongée (3371).

4. Élément de contact avec un patient (3370) selon la revendication 2 ou la revendication 3, dans lequel le ou chaque moyen de réception comprend une patte (3320) pourvue d'une fente (3321) servant à recevoir la sangle (3310).

5. Élément de contact avec un patient (3370) selon l'une quelconque des revendications 2 à 4, dans lequel l'élément de contact avec un patient (3370) comprend une sangle supplémentaire ou une partie de sangle (3318), la sangle supplémentaire ou la partie de sangle (3318) étant configurée pour passer autour de l'arrière du cou du patient, en utilisation.

6. Élément de contact avec un patient (3370) selon l'une quelconque des revendications 1 à 5, dans lequel la partie manchon allongée (3371) comprend une partie rigidifiée.

7. Élément de contact avec un patient (3370) selon la revendication 6, dans lequel la partie rigidifiée est courbée.

8. Élément de contact avec un patient (3370) selon l'une quelconque des revendications 1 à 7, dans lequel la partie fenêtre (3373) comprend un premier côté et un second côté opposé, et dans lequel l'élément de recouvrement comprend un cordon élastique (3376, 3377) qui coopère avec le premier côté et avec le second côté.

9. Élément de contact avec un patient (3370) selon la revendication 8, dans lequel le premier côté est pourvu d'une pluralité de premières boucles (3375, 3375A) et le second côté est pourvu d'une pluralité de secondes boucles (3375, 3375B), dans lequel le cordon élastique (3376, 3377) s'étend à travers une pluralité des premières boucles (3375, 3375A) et à travers une pluralité des secondes boucles (3375, 3375B).

10. Élément de contact avec un patient (3370) selon l'une quelconque des revendications 1 à 7, dans lequel l'élément de recouvrement comprend un treillis (3374).

11. Élément de contact avec un patient (3370) selon l'une quelconque des revendications 1 à 7, dans lequel l'élément de recouvrement comprend un matériau transparent.
